# EUROPEAN PATENT APPLICATION

(11) **EP 4 464 784 A1**
(43) Date of publication of application: **20.11.2024**
(21) Application number: 23740467.8
(22) Date of filing: 12.01.2023
(51) Int. Cl.: C12N 15/86, C12N 5/079, C12N 15/90

(54) **SCHWANN CELL-SPECIFIC PROMOTER**

(30) Priority: 13.01.2022 KR 20220005303
(71) Applicant: Toolgen Incorporated, Seoul 07789 (KR)
(72) Inventor: SONG, Dong Woo, Seoul 07789 (KR); LEE, Hyerim, Seoul 07789 (KR); OH, Hye-Kyung, Seoul 07789 (KR); CHOI, Beom Seok, Seoul 07789 (KR); LEE, Kyu Jun, Seoul 07789 (KR); GO, Nan Yeong, Seoul 07789 (KR); LEE, Jae Young, Seoul 07789 (KR)
(74) Representative: dompatent von Kreisler Selting Werner - Partnerschaft von Patent- und Rechtsanwälten mbB
(86) International application number: PCT/KR2023/000569
(87) International publication number: WO 2023/136624

(57) **Abstract**

The present invention relates to a Schwann cell-specific promoter and use thereof. More particularly, the present invention relates to an artificially engineered minimal promoter that specifically operates in Schwann cells, wherein the artificially engineered minimal promoter is derived from an MPZ (Myelin Protein Zero or P0) promoter. In addition, the present invention relates to a vector using the Schwann cell-specific promoter and a method for treating Schwann cell-related diseases using same.

## Description

### Technical Field

The present disclosure relates to a Schwann cell-specific promoter and uses thereof. More specifically, the present disclosure relates to an artificially engineered minimal promoter that specifically operates in Schwann cells. The artificially engineered minimal promoter is derived from the promoter of Myelin Protein Zero (MPZ or P0). Additionally, the present disclosure relates to a vector using the Schwann cell-specific promoter and to a method of treating diseases related to Schwann cells using the same.

### Background Art

Schwann cells (hereinafter abbreviated as SC) or neurolemmocytes are the basic glial cells of the peripheral nervous system. Glial cells support neurons. In axons with myelin, Schwann cells form a myelin sheath. The nervous system of vertebrates is insulated by the myelin sheath, which maintains the membrane capacitance of axons.

Charcot-Marie-Tooth (CMT) disease is a genetic disease. Charcot-Marie-Tooth (CMT) disease causes a gradual weakening of the muscles in the hands and feet. This happens because of abnormalities in the Schwann cells responsible for forming the myelin sheath in the peripheral nervous system. Among the types of CMT disease, Charcot-Marie-Tooth type 1A (CMT1A) is one of the most frequently occurring genetic diseases in the peripheral nervous system.

CMT1A accounts for more than half of all CMT cases and approximately 70% of CMT1 cases, and the incidence of CMT is 1/2500. CMT1A causes pathological properties such as muscle weakness and loss, decreased reflexes, peripheral sensory impairment, deformities of the hands and feet, slowing of nerve conduction velocity (NCV), and hypertrophic segmental demyelination and remyelination, which involves onion bulb formation.

Recently, gene therapy technology has been applied to diseases caused by Schwann cell dysfunction such as CMT1A. In addition to CMT disease, gene therapy technology targeting Schwann cells may be applied to many other diseases related to Schwann cells such as motor neuron disease (MND), and may also be applied to diseases caused by factors other than genetic factors. The causes of most of these diseases are diverse and poorly understood, so targeting these diseases using viral vectors may be beneficial. To improve therapeutic outcomes for Schwann cell-associated diseases, including demyelinating neuropathies like CMT, there is still a need for enhanced methods to target these conditions.

### Disclosure

### Technical Problem

One objective of the present disclosure is to provide a Schwann cell-specific promoter.

Another objective of the present disclosure is to provide a Schwann cell-specific expression vector.

Yet another objective of the present disclosure is to provide a method of treating diseases related to Schwann cells using the Schwann cell-specific expression vector.

### Technical Solution

The present disclosure provides a Schwann cell-specific promoter.

The Schwann cell-specific promoter may include a sequence selected from:

SEQ ID NO: 3; SEQ ID NO: 4; SEQ ID NO: 8; SEQ ID NO: 9; SEQ ID NO: 13; SEQ ID NO: 14; and a sequence having at least 70% homology with one of SEQ ID NOs: 3, 4, 8, 9, 13, and 14.

The present disclosure provides a Schwann cell-specific expression vector.

The Schwann cell-specific expression vector may include a Schwann cell-specific promoter; and a gene of interest.

The gene of interest may be operably linked to the Schwann cell-specific promoter.

The Schwann cell-specific promoter may include a sequence selected from: SEQ ID NO: 3; SEQ ID NO: 4; SEQ ID NO: 8; SEQ ID NO: 9; SEQ ID NO: 13; SEQ ID NO: 14; and a sequence having at least 70% homology with one of SEQ ID NOs: 3, 4, 8, 9, 13, and 14.

The gene of interest may be a gene configured to be expressed in Schwann cells or a nucleic acid encoding a protein to be expressed in Schwann cells. Herein, the gene of interest may be a nucleic acid encoding a protein used for artificially engineering genes in Schwann cells. For example, the protein for artificially engineering genes may be a Cas protein, zinc finger nuclease (ZFN), or tranion activator-like effector nuclease (TALEN). Herein, the Cas protein may be a Cas9 protein, Cas12a1 (Cpf1) protein, or Cas12f1 protein. For example, the Cas9 protein may be a *Streptococcus pyogenes* Cas9 (SpCas9) protein, *Staphylococcus aureus* Cas9 (SaCas9) protein, *Campylobacter jejuni* Cas9 (CjCas9) protein, or *Staphylococcus auricularis* Cas9 (SauriCas9) protein. Herein, a gene encoding the protein for artificially engineering genes may be expressed to edit genes associated with diseases related to Schwann cells. By using this, the protein can induce a knock-out effect on genes associated with diseases related to Schwann cells.

In addition, the gene of interest serves as a gene associated with diseases related to Schwann cells, so, if necessary, the promoter of the present disclosure can be used to overexpress the gene of interest in Schwann cells.

In one example, the gene associated with diseases related to Schwann cells may be a connexin 32 (Cx32) gene, SH3 domain and tetratricopeptide repeats 2 (SH3TC2) gene, myelin protein zero (MPZ) gene, early growth response 2 (EGR2) gene, ganglioside induced differentiation associated protein 1 (GDAP1) gene, N-Myc downstream regulated 1 (NDRG1) gene, or peripheral myelin protein 22 (PMP22) gene. To obtain disease treatment effects, the genes can be knocked out or overexpressed if needed.

The Schwann cell-specific expression vector may include a viral vector or non-viral vector. Herein, the viral vector may be one or more viral vectors selected from the group consisting of a retroviral (retrovirus) vector, lentiviral (lentivirus) vector, adenoviral (adenovirus) vector, adeno-associated viral (adeno-associated virus; AAV) vector, vaccinia viral (vaccinia virus) vector, poxviral (poxvirus) vector, and herpes simplex viral (herpes simplex virus) vector.

The Schwann cell-specific expression vector may further comprise one or more regulatory/control element. Herein, the regulatory/control element may be an enhancer, artificial intron, polyadenylation signal, and/or WPRE. In another example, the Schwann cell-specific expression vector may include the polyadenylation signal. Herein, the polyadenylation signal may be a synpA or SV40pA. In yet another example, the Schwann cell-specific expression vector may comprise the artificial intron and the WPRE. Herein the artificial intron may be an MVM intron, and the WPRE may be a WPRE3. In yet another example, the Schwann cell-specific expression vector may comprise the artificial intron, WPRE, and polyadenylation signal. Herein the artificial intron may be the MVM intron, the WPRE may be the WPRE3, and the polyadenylation signal may be the synpA or SV40pA.

The present disclosure provides a composition comprising a Schwann cell-specific expression vector.

The composition may comprise the Schwann cell-specific expression vector described above.

The present disclosure provides a method of treating diseases associated with Schwann cells.

The method may comprise introducing (or delivering) a composition to a subject.

The composition may comprise the Schwann cell-specific expression vector. Herein, the Schwann cell-specific expression vector is as described above.

The subject may be a human or non-human animal with diseases associated with Schwann cells.

### Advantageous Effects

The present disclosure relates to a Schwann cell-specific promoter and uses thereof. Through the technology disclosed herein, a vector including the Schwann cell-specific promoter and uses thereof can be provided. In addition, a pharmaceutical composition and a method for treating diseases related to Schwann cells can be provided using the vector including the Schwann cell-specific promoter.

### Brief Description of The Drawings

Fig. 1 is a graph showing the expression of Cas9 by engineered MPZ promoter in Rat schwann cell line.
Fig. 2 is a graph showing the gene editing efficiency for targeting pmp22-TATA by the expression of Cas9 under control of engineered MPZ promoter in Rat schwann cell line.
Fig. 3 is a graph showing the expression of Cas9 by engineered MPZ promoter in Human schwann cell line.
Fig. 4 is a graph showing the gene editing efficiency for targeting pmp22-TATA by the expression of Cas9 under control of engineered MPZ promoter and the expression of optimized sgRNA vector, in S16 cells.
Fig. 5 is a graph showing the gene editing efficiency for targeting pmp22-TATA by the expression of Cas9 under control of engineered MPZ promoter and the expression of optimized sgRNA vector, in RT4 cells.
Fig. 6 is a graph showing the gene editing efficiency for targeting pmp22-TATA by the expression of Cas9 by other polyA vector and engineered MPZ promoter in S16 cells.
Fig. 7 is a graph showing the gene editing efficiency for targeting pmp22-TATA by combination of small SaurCas9 capable of single AAV packaging, optimized element (Intron, WPRE), and engineered MPZ promoter in RT4 cells.
Fig. 8 is a graph showing the gene editing efficiency for targeting pmp22-TATA by the expression of Cas9 under control of engineered MPZ promoter in RT4-D6P2T cell line.
Fig. 9 is a graph showing the gene editing efficiency for targeting pmp22-TATA by the expression of Cas9 under control of engineered MPZ promoter in RT4-D6P2T cell line and using sgRNAs with different sequences (Table 1).
Fig. 10 is a graph showing the expression of tdTomato by engineered MPZ promoter in RT4-D6P2T cell line.
Fig. 11 is a graph showing the level of transformation of pAAV-tdTomato-related vector into RT4-D6P2T cell lines
Figs. 12 and 13 are graphs showing the gene editing efficiency (indels) in the sciatic nerve, lumbar nerve, and liver of rats after co-transfection with vectors expressing sgRNA targeting the pmp22-TATA region (Table 1), along with vectors expressing SpCas9 under the control of the EFS promoter and vectors expressing SpCas9 under the control of the IE200 promoter into the tail vein of the rats.

### Best Mode

### Term definition

The definitions of terms used in this specification are as follows.

### About

As used herein, the term "about" means quantity, level, value, number, frequency, percentage, dimension, size, amount, weight, or length that varies by 30%, 25%, 20%, 15%, 10%, 9%, 8%, 7%, 6%, 5%, 4%, 3%, 2%, or 1% based on reference quantity, level, value, number, frequency, percentage, dimension, size, amount, weight, or length.

### Vector

The term "Vector", unless otherwise specified, refers collectively to all substances capable of transporting genetic material into a cell. For example, the vector may be a non-viral vector or a viral vector including a genetic material, that is, a gene of interest or a nucleic acid of interest to be expressed in a cell, in more detail, a nucleic acid encoding the Cas protein of the CRISPR/Cas system or a nucleic acid encoding a protein involved in the function of Schwann cells, but the vector is not limited thereto. The terms include all meanings recognized by those skilled in the art and may be appropriately interpreted depending on the context.

### Guide RNA

The term "Guide RNA" refers to RNA that has the function of inducing the CRISPR/Cas9 complex to recognize a specific sequence included in a target nucleic acid. The composition of the guide RNA may be broadly divided functionally into 1) a scaffold portion, and 2) a guide domain portion. The scaffold portion is a portion that interacts with the Cas9 protein and binds with the Cas9 protein to form a complex. The scaffold portion is determined by the type of microorganism from which the Cas9 protein is derived. The guide domain portion is a portion that may bind complementary to a nucleotide sequence portion of a predetermined length in the target nucleic acid. The guide domain portion is a sequence that may be artificially engineered and is determined by the target nucleotide sequence of interest. In addition, the terms include all meanings recognized by those skilled in the art and may be appropriately interpreted depending on the context.

### Operably linked

The term "operably linked" means that a specific component is arranged in a functional relationship with another component, such that the specific component is connected to the other component in a manner that allows the specific component to function as intended. For example, when a promoter sequence is operably linked to a sequence encoding a protein A, the term means that the promoter is linked to the sequence encoding the protein A to transcribe and/or express the sequence encoding the protein A in the cell. In addition, the terms include all meanings recognized by those skilled in the art and may be appropriately interpreted depending on the context.

### Engineered

The term "engineered" or "artificially engineered" is used to distinguish engineered or artificially engineered substances and molecules from substances and molecules, the composition of which already exists in nature. The term means that artificial modifications have been made to substances and molecules, the composition of which already exists in nature. For example, "artificially engineered MPZ promoter" refers to an MPZ promoter obtained by artificially modifying an MPZ promoter that exists in nature. In this case, some sequences of the MPZ promoter that exist in nature may be deleted and/or substituted with other sequences, but the engineering is not limited thereto. In addition, the terms include all meanings recognized by those skilled in the art and may be appropriately interpreted depending on the context.

Unless otherwise defined, all technical and scientific terms used in this specification have the same meaning as commonly understood by those skilled in the art to which the present disclosure pertains. Although methods and materials similar or equivalent to those described herein may be used in the practice or testing of the present disclosure, suitable methods and materials are described below. All publications, patents, and other references mentioned herein are incorporated by reference in their entirety. Additionally, the materials, methods, examples, and experiments are illustrative only and are not intended to be limiting.

Hereinafter, the present disclosure will be described.

Recently, gene therapy technology has been developed to treat diseases caused by Schwann cell dysfunction such as CMT1A. For this gene therapy, a vector system such as a viral vector is used. The main feature of the vector system is the inclusion of a promoter that specifically expresses in Schwann cells.

Currently, various researchers are developing Schwann cell-specific promoters. In particular, an MPZ promoter, which specifically expresses in Schwann cells, is used. Kleopas A. Kleopa et al. (International Patent Application Publication WO2020/245169) developed a viral vector including a GJB1 gene operably linked to the MPZ promoter or mini-MPZ promoter to treat CMT diseases. At this time, as the MPZ promoter, a full-length promoter of 1.2 kb was used, or a mini-MPZ promoter including a partial sequence of 410 bp present upstream of the start codon of the MPZ gene was used. In this way, the MPZ promoter is used as the Schwann cell-specific promoter.

In general, in the case of gene therapy using a viral vector system, the capacity of the viral vector system is limited. The capacity may be in a range of at least 2 kb to 50 kb depending on the virus packaging capacity and may vary depending on the type of virus. Therefore, to effectively use limited capacity, it is necessary to use a small-sized promoter.

Accordingly, the present inventors used the MPZ promoter to develop a Schwann cell-specific promoter that shows higher expression efficiency specifically for Schwann cells and has a smaller size.

### 1. Schwann cell-specific promoter

In one aspect, the present disclosure relates to a Schwann cell-specific promoter. The Schwann cell-specific promoter is a promoter that operates specifically in Schwann cells. This means that when the Schwann cell-specific promoter is used, the expression of the gene of interest (for example, a nucleic acid encoding a gene or protein of interest operably linked to the Schwann cell-specific promoter) is increased in Schwann cells by the Schwann cell-specific promoter compared to in other cells. The Schwann cell-specific promoter, as described above, is derived from the MPZ promoter. In particular, the Schwann cell-specific promoter is an artificially engineered promoter derived from the MPZ promoter. At this time, the MPZ promoter may be the promoter of a human or rat MPZ gene. The promoter of the rat MPZ gene (hereinafter referred to as rat MPZ promoter) has the following nucleic acid sequence:

GTTCCAGAAAACACCACTCAGTTCCTTGTCCCCCGCTCTCTCCACCCCACAGACGCTCTGGGC
C-3′(SEQ ID NO: 1).

The promoter of the human MPZ gene (hereinafter referred to as human MPZ promoter) has the following nucleic acid sequence:

5′-
ACTATGCCTGGCATAAACTTCATTTATTAAAGTTTATTTTGTCTTTAATCTCTCATATAACTT
AGTCTTCCTGATATTGCAGCTGTGTGTGCCCCTCTTTTGTACTCCCAGCATTTTGTTCATTAC
TAAAGGAAGTGTCATGGCTTATTATACTTGATTGTTGATGGGTTTGTCCTCTGATCTTCCCAT
CTCCACCTCCCCAAACCAAATTTTCAACTCCTTGCTGGAAGGACTTAATTTTTATTCCTCTCT
CTATTACCTGCATTCTCATACTTTACATATTGCTGGCACTTAATACAATTTTGTAGCCTTGAA
ATAAATTGAAATGGACTTAAACAGCAGCATGAAGCACTGAAGGACTTCTTGACAAACGGAAAG
GTCAGGGGCTTCTTGCCTGGAAATAGTCCAGTGGAGAAAAACTTCTGTCTGGGAAGAATCGCA
CAGGATGAAGGGAGGTGCGGGGAAAAAAACTCCCATAGGACTTGGTCATCTCAAGAAGTCTGT
AATGCAGCCCACATTAGAGGAGATAACAGGGGATATCCTATTTTCAGAGTTCTCTGGGGGAAA
CCTCCCTCTAGTTCCTAGGGCTGTGAGGCAGCCTCTCTCAGGCAAGGAGGCTGAGGAGAAATC
CCTTTTTATGGCCTTTAAATTGAGGTTCCATATCTATCCCTCAGAGAAGTGTGTCTGTGTCCC
TGTTTTTGTCCCTCTCCCTCACCACCCCCCACAACATTCCAGCCTGGGGCAGGGGGAGGCCAG
TGGACACAAAGCCCTCTGTGTATGGGGTGGTATGTGTCCCCCCACCCCTCCACCCAGACTATA
CAATGCCCCTTCTGCTCCCTGCACTCTGCCCCCCTCCCCACCACCTCTCAACTGCACATGCCA
GGCTGCAATTGGTTACTGGCTGAGGACAGCCCCCTCATGCTGGGGCCCTAGGGGATTTTAAGC
AGGTTCCAGGAACCCCCCGTTCAGTTCCTGGTCCCCCACTTTCTCAACCCCACAG-3′(SEQ
ID NO: 2).

More specifically, the Schwann cell-specific promoter may be an artificially engineered MPZ promoter in which some nucleotide sequences of the MPZ promoter have been deleted.

The Schwann cell-specific promoter may be an artificially engineered MPZ promoter in which some nucleotide sequences at the 5' end of the MPZ promoter have been deleted. At this time, the deleted nucleotide sequences at the 5' end of the MPZ promoter may be a nucleotide sequence with a length in a range of 1 to 100 bp, 100 to 200 bp, 200 to 300 bp, 300 to 400 bp, 400 to 500 bp, 500 to 600 bp, 600 to 700 bp, 700 to 800 bp, or 800 to 900 bp. At this time, the artificially engineered MPZ promoter may comprise a sequence length in a range of 100 to 1000 bp, 100 to 900 bp, 100 to 800 bp, 100 to 700 bp, 100 to 600 bp, 100 to 500 bp, 100 to 400 bp, 100 to 300 bp, or 100 to 200 bp.

The Schwann cell-specific promoter may be an artificially engineered MPZ promoter in which some nucleotide sequences at the 3' end of the MPZ promoter have been deleted. At this time, the deleted nucleotide sequences at the 3' end of the MPZ promoter may be a nucleotide sequence with a length in a range of 1 to 100 bp, 100 to 200 bp, 200 to 300 bp, 300 to 400 bp, 400 to 500 bp, 500 to 600 bp, 600 to 700 bp, 700 to 800 bp, or 800 to 900 bp. At this time, the artificially engineered MPZ promoter may comprise a sequence length in a range of 100 to 1000 bp, 100 to 900 bp, 100 to 800 bp, 100 to 700 bp, 100 to 600 bp, 100 to 500 bp, 100 to 400 bp, 100 to 300 bp, or 100 to 200 bp.

The Schwann cell-specific promoter may include a TATA box or CAAT box.

The Schwann cell-specific promoter may include a transcription start site (TSS).

The Schwann cell-specific promoter may further include an intronic enhancer (IE) at the 5' end. At this time, the IE may include Egr2 and Sox10 binding sites. The IE may be a region including the Egr2 and Sox10 binding sites present in the intron 1 region of the MPZ gene. The IE may have a sequence length in a range of 1 to 100 bp, 100 to 200 bp, 200 to 300 bp, or 300 to 400 bp.

The Schwann cell-specific promoter may further include an intronic enhancer (IE) at the 3' end. At this time, the IE may include Egr2 and Sox10 binding sites. The IE may be a region including the Egr2 and Sox10 binding sites present in the intron 1 region of the MPZ gene. The IE may have a sequence length in a range of 1 to 100 bp, 100 to 200 bp, 200 to 300 bp, or 300 to 400 bp.

### 1-1. Schwann cell-specific promoter (1)

In one embodiment, the Schwann cell-specific promoter may be an artificially engineered rat MPZ promoter in which some nucleotide sequences of the rat MPZ promoter have been deleted.

The Schwann cell-specific promoter may be an artificially engineered rat MPZ promoter in which some nucleotide sequences at the 5' end of the rat MPZ promoter have been deleted. At this time, the deleted nucleotide sequences at the 5' end of the MPZ promoter may be a nucleotide sequence with a length in a range of 1 to 100 bp, 100 to 200 bp, 200 to 300 bp, 300 to 400 bp, 400 to 500 bp, 500 to 600 bp, 600 to 700 bp, 700 to 800 bp, or 800 to 900 bp. Preferably, the deleted nucleotide sequences at the 5' end of the MPZ promoter may be a nucleotide sequence with a length in a range of 800 to 900 bp. At this time, the artificially engineered rat MPZ promoter may have a sequence length in a range of 100 to 1000 bp, 100 to 900 bp, 100 to 800 bp, 100 to 700 bp, 100 to 600 bp, 100 to 500 bp, 100 to 400 bp, 100 to 300 bp, or 100 to 200 bp. Preferably, the artificially engineered rat MPZ promoter may consist of a sequence length in a range of 200 to 300 bp.

The Schwann cell-specific promoter may include a TATA box or CAAT box.

The Schwann cell-specific promoter may include a transcription start site (TSS).

In another embodiment, the Schwann cell-specific promoter may comprise the nucleotide sequence shown in SEQ ID NO: 3 with a length of 300 bp: 5'-GCCTCACCCCACCCCAACATTCCAACCTAGGGTAGGGGGAGGTCAGTATACACAAAGCCCTCT GTGTAAGGGGTGGTATGTGTCCCCCCACCCCCCTACCCAGAGTATACAATGCCCCTTCTGCTC CATGCCCCTGCCACCCTCCCCACCACCTCTCAATTGCACATGCCAGGCTGCAATTGGTCACTG GCTCAGGACAGCCCCCTCATGCTGGGGATCCAGGGGATTTTAAGCAGGTTCCAGAAAACACCA CTCAGTTCCTTGTCCCCCGCTCTCTCCACCCCACAGACGCTCTGGGCC-3' (SEQ ID NO: 3). Alternatively, the Schwann cell-specific promoter may comprise a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 3.

In yet another embodiment, the Schwann cell-specific promoter may comprise the nucleotide sequence shown in SEQ ID NO: 4 with a length of 200 bp: 5'-CAGAGTATACAATGCCCCTTCTGCTCCATGCCCCTGCCACCCTCCCCACCACCTCTCAATTGC ACATGCCAGGCTGCAATTGGTCACTGGCTCAGGACAGCCCCCTCATGCTGGGGATCCAGGGGA TTTTAAGCAGGTTCCAGAAAACACCACTCAGTTCCTTGTCCCCCGCTCTCTCCACCCCACAGA CGCTCTGGGCC-3' (SEQ ID NO: 4). Alternatively, the Schwann cell-specific promoter may comprise a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 4.

In yet another embodiment, the Schwann cell-specific promoter may be an artificially engineered human MPZ promoter in which some nucleotide sequences of the human MPZ promoter have been deleted.

The Schwann cell-specific promoter may be an artificially engineered human MPZ promoter in which some nucleotide sequences at the 5' end of the human MPZ promoter have been deleted. At this time, the deleted nucleotide sequences at the 5' end of the MPZ promoter may be a nucleotide sequence with a length in a range of 1 to 100 bp, 100 to 200 bp, 200 to 300 bp, 300 to 400 bp, 400 to 500 bp, 500 to 600 bp, 600 to 700 bp, 700 to 800 bp, or 800 to 900 bp. Preferably, the deleted nucleotide sequences at the 5' end of the MPZ promoter may be a nucleotide sequence with a length in a range of 800 to 900 bp. At this time, the artificially engineered human MPZ promoter may have a sequence length in a range of 100 to 1000 bp, 100 to 900 bp, 100 to 800 bp, 100 to 700 bp, 100 to 600 bp, 100 to 500 bp, 100 to 400 bp, 100 to 300 bp, or 100 to 200 bp. Preferably, the artificially engineered human MPZ promoter may have a sequence length in a range of 200 to 300 bp.

The Schwann cell-specific promoter may include a TATA box or CAAT box.

The Schwann cell-specific promoter may include a transcription start site (TSS).

In yet another embodiment, the Schwann cell-specific promoter may comprise the nucleotide sequence shown in SEQ ID NO: 5 with a length of 300 bp: 5'-GTCCCTCTCCCTCACCACCCCCCACAACATTCCAGCCTGGGGCAGGGGGAGGCCAGTGGACAC AAAGCCCTCTGTGTATGGGGTGGTATGTGTCCCCCCACCCCTCCACCCAGACTATACAATGCC CCTTCTGCTCCCTGCACTCTGCCCCCCTCCCCACCACCTCTCAACTGCACATGCCAGGCTGCA ATTGGTTACTGGCTGAGGACAGCCCCCTCATGCTGGGGCCCTAGGGGATTTTAAGCAGGTTCC AGGAACCCCCCGTTCAGTTCCTGGTCCCCCACTTTCTCAACCCCACAG-3' (SEQ ID NO: 5). Alternatively, the Schwann cell-specific promoter may comprise a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 5.

In yet another embodiment, the Schwann cell-specific promoter may comprise the nucleotide sequence shown in SEQ ID NO: 6 with a length of 200 bp: 5'-CCCCTCCACCCAGACTATACAATGCCCCTTCTGCTCCCTGCACTCTGCCCCCCTCCCCACCAC CTCTCAACTGCACATGCCAGGCTGCAATTGGTTACTGGCTGAGGACAGCCCCCTCATGCTGGG GCCCTAGGGGATTTTAAGCAGGTTCCAGGAACCCCCCGTTCAGTTCCTGGTCCCCCACTTTCT CAACCCCACAG-3' (SEQ ID NO: 6). Alternatively, the Schwann cell-specific promoter may comprise a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 6.

### 1-2. Schwann cell-specific promoter (2)

In one embodiment, the Schwann cell-specific promoter comprises the artificially engineered rat MPZ promoter in which some nucleotide sequences of the rat MPZ promoter have been deleted and comprises the IE which is positioned at the 5' end of the artificially engineered rat MPZ promoter.

The Schwann cell-specific promoter may be an artificially engineered rat MPZ promoter in which some nucleotide sequences at the 5' end of the rat MPZ promoter have been deleted. At this time, the deleted nucleotide sequences at the 5' end of the MPZ promoter may be a nucleotide sequence with a length in a range of 1 to 100 bp, 100 to 200 bp, 200 to 300 bp, 300 to 400 bp, 400 to 500 bp, 500 to 600 bp, 600 to 700 bp, 700 to 800 bp, or 800 to 900 bp. Preferably, the deleted nucleotide sequences at the 5' end of the MPZ promoter may be a nucleotide sequence with a length in a range of 800 to 900 bp. At this time, the artificially engineered rat MPZ promoter may comprise a sequence length in a range of 100 to 1000 bp, 100 to 900 bp, 100 to 800 bp, 100 to 700 bp, 100 to 600 bp, 100 to 500 bp, 100 to 400 bp, 100 to 300 bp, or 100 to 200 bp. Preferably, the artificially engineered rat MPZ promoter may comprise a sequence length in a range of 200 to 300 bp.

The Schwann cell-specific promoter may include a TATA box or CAAT box.

The Schwann cell-specific promoter may include a transcription start site (TSS).

The IE may include Egr2 and Sox10 binding site. The IE may be a region including the Egr2 and Sox10 binding site present in the intron 1 region of the MPZ gene. The IE may have a sequence length in a range of 1 to 100 bp, 100 to 200 bp, 200 to 300 bp, or 300 to 400 bp. Preferably, the IE may have a sequence length in a range of 100 to 200 bp, and more preferably, the IE may have a sequence length of 117 bp. In another embodiment, the IE may comprise the nucleotide sequence shown in SEQ ID NO: 7 with a length of 117 bp: 5'-GACAATGAGACTTTGTTTGGTCGCCTCCTCCTAAAGAACAGAAAAGTCTATAATTGTTCCTCC CCAGAGCCAGCCCACACACATAGACTGCCTGGGATGACTCTCCCTGTGTGGGCG-3' (SEQ ID NO: 7). Alternatively, the IE may comprise a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 7.

In another embodiment, the Schwann cell-specific promoter may be in a form where the IE, which has the nucleotide sequence shown in SEQ ID NO: 7 with a length of 117 bp, is connected to the 5' end of the artificially engineered rat MPZ promoter, which has the nucleotide sequence shown in SEQ ID NO: 3 with a length of 300 bp, and the Schwann cell-specific promoter may comprise a nucleotide sequence shown in SEQ ID NO: 8: 5'-GACAATGAGACTTTGTTTGGTCGCCTCCTCCTAAAGAACAGAAAAGTCTATAATTGTTCCTCC CCAGAGCCAGCCCACACACATAGACTGCCTGGGATGACTCTCCCTGTGTGGGCGGCCTCACCC CACCCCAACATTCCAACCTAGGGTAGGGGGAGGTCAGTATACACAAAGCCCTCTGTGTAAGGG GTGGTATGTGTCCCCCCACCCCCCTACCCAGAGTATACAATGCCCCTTCTGCTCCATGCCCCT GCCACCCTCCCCACCACCTCTCAATTGCACATGCCAGGCTGCAATTGGTCACTGGCTCAGGAC AGCCCCCTCATGCTGGGGATCCAGGGGATTTTAAGCAGGTTCCAGAAAACACCACTCAGTTCC TTGTCCCCCGCTCTCTCCACCCCACAGACGCTCTGGGCC-3' (SEQ ID NO: 8). At this time, the Schwann cell-specific promoter may have a sequence length of 417 bp. Alternatively, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 8.

In another embodiment, the Schwann cell-specific promoter may be in a form where the IE, which has the nucleotide sequence shown in SEQ ID NO: 7 with a length of 117 bp, is connected to the 5' end of the artificially engineered rat MPZ promoter, which has the nucleotide sequence shown in SEQ ID NO: 4 with a length of 200 bp, and the Schwann cell-specific promoter may comprise a nucleotide sequence shown in SEQ ID NO: 9: 5'-GACAATGAGACTTTGTTTGGTCGCCTCCTCCTAAAGAACAGAAAAGTCTATAATTGTTCCTCC CCAGAGCCAGCCCACACACATAGACTGCCTGGGATGACTCTCCCTGTGTGGGCGCAGAGTATA CAATGCCCCTTCTGCTCCATGCCCCTGCCACCCTCCCCACCACCTCTCAATTGCACATGCCAG GCTGCAATTGGTCACTGGCTCAGGACAGCCCCCTCATGCTGGGGATCCAGGGGATTTTAAGCA GGTTCCAGAAAACACCACTCAGTTCCTTGTCCCCCGCTCTCTCCACCCCACAGACGCTCTGGG CC-3' (SEQ ID NO: 9). At this time, the Schwann cell-specific promoter may have a sequence length of 317 bp. Alternatively, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 9.

In another embodiment, the Schwann cell-specific promoter comprises the artificially engineered human MPZ promoter in which some nucleotide sequences of the human MPZ promoter have been deleted and comprises the IE which is positioned at the 5' end of the artificially engineered human MPZ promoter.

The Schwann cell-specific promoter may be an artificially engineered human MPZ promoter in which some nucleotide sequences at the 5' end of the human MPZ promoter have been deleted. At this time, the deleted nucleotide sequences at the 5' end of the MPZ promoter may be a nucleotide sequence with a length in a range of 1 to 100 bp, 100 to 200 bp, 200 to 300 bp, 300 to 400 bp, 400 to 500 bp, 500 to 600 bp, 600 to 700 bp, 700 to 800 bp, or 800 to 900 bp. Preferably, the deleted nucleotide sequences at the 5' end of the MPZ promoter may be a nucleotide sequence with a length in a range of 800 to 900 bp. At this time, the artificially engineered human MPZ promoter may have a sequence length in a range of 100 to 1000 bp, 100 to 900 bp, 100 to 800 bp, 100 to 700 bp, 100 to 600 bp, 100 to 500 bp, 100 to 400 bp, 100 to 300 bp, or 100 to 200 bp. Preferably, the artificially engineered human MPZ promoter may have a sequence length in a range of 200 to 300 bp.

The Schwann cell-specific promoter may include a TATA box or CAAT box.

The Schwann cell-specific promoter may include a transcription start site (TSS).

The IE may include Egr2 and Sox10 binding site. The IE may be a region including the Egr2 and Sox10 binding site present in the intron 1 region of the MPZ gene. The IE may have a sequence length in a range of 1 to 100 bp, 100 to 200 bp, 200 to 300 bp, or 300 to 400 bp. Preferably, the IE may have a sequence length in a range of 100 to 200 bp, and more preferably, the IE may have a sequence length of 123 bp. In yet another embodiment, the IE may comprise the nucleotide sequence shown in SEQ ID NO: 10 with a length of 123 bp: 5'-GACAATGGAACTTTGTTTGGTTGCCACCTCCCCCTCCTGTAGAACAAAAAGGTCTACAGTTGC CCCTCCCTGGGGCCAGCCCACACACATAGTCTCTCTGGAATGACCTTCCTTGTGTGGGTA-3' (SEQ ID NO: 10). Alternatively, the IE may comprise a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 10.

In another embodiment, the Schwann cell-specific promoter may be in a form where the IE, which has the nucleotide sequence shown in SEQ ID NO: 10 with a length of 123 bp, is connected to the 5' end of the artificially engineered human MPZ promoter, which has the nucleotide sequence shown in SEQ ID NO: 5 with a length of 300 bp, and the Schwann cell-specific promoter may comprise a nucleotide sequence shown in SEQ ID NO: 11: 5'-GACAATGGAACTTTGTTTGGTTGCCACCTCCCCCTCCTGTAGAACAAAAAGGTCTACAGTTGC CCCTCCCTGGGGCCAGCCCACACACATAGTCTCTCTGGAATGACCTTCCTTGTGTGGGTAGTC CCTCTCCCTCACCACCCCCCACAACATTCCAGCCTGGGGCAGGGGGAGGCCAGTGGACACAAA GCCCTCTGTGTATGGGGTGGTATGTGTCCCCCCACCCCTCCACCCAGACTATACAATGCCCCT TCTGCTCCCTGCACTCTGCCCCCCTCCCCACCACCTCTCAACTGCACATGCCAGGCTGCAATT GGTTACTGGCTGAGGACAGCCCCCTCATGCTGGGGCCCTAGGGGATTTTAAGCAGGTTCCAGG AACCCCCCGTTCAGTTCCTGGTCCCCCACTTTCTCAACCCCACAG-3' (SEQ ID NO: 11). At this time, the Schwann cell-specific promoter may have a sequence length of 423 bp. Alternatively, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 11.

In another embodiment, the Schwann cell-specific promoter may be in a form where the IE, which has the nucleotide sequence shown in SEQ ID NO: 10 with a length of 123 bp, is connected to the 5' end of the artificially engineered human MPZ promoter, which has the nucleotide sequence shown in SEQ ID NO: 6 with a length of 200 bp, and the Schwann cell-specific promoter may comprise a nucleotide sequence shown in SEQ ID NO: 12: 5'-GACAATGGAACTTTGTTTGGTTGCCACCTCCCCCTCCTGTAGAACAAAAAGGTCTACAGTTGC CCCTCCCTGGGGCCAGCCCACACACATAGTCTCTCTGGAATGACCTTCCTTGTGTGGGTACCC CTCCACCCAGACTATACAATGCCCCTTCTGCTCCCTGCACTCTGCCCCCCTCCCCACCACCTC TCAACTGCACATGCCAGGCTGCAATTGGTTACTGGCTGAGGACAGCCCCCTCATGCTGGGGCC CTAGGGGATTTTAAGCAGGTTCCAGGAACCCCCCGTTCAGTTCCTGGTCCCCCACTTTCTCAA CCCCACAG-3' (SEQ ID NO: 12). At this time, the Schwann cell-specific promoter may comprise a sequence length of 323 bp. Alternatively, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 12.

### 1-3. Schwann cell-specific promoter (3)

In one embodiment, the Schwann cell-specific promoter comprises the artificially engineered rat MPZ promoter in which some nucleotide sequences of the rat MPZ promoter have been deleted and comprises the IE which is positioned at the 3' end of the artificially engineered rat MPZ promoter.

The Schwann cell-specific promoter may be an artificially engineered rat MPZ promoter in which some nucleotide sequences at the 5' end of the rat MPZ promoter have been deleted. At this time, the deleted nucleotide sequences at the 5' end of the MPZ promoter may be a nucleotide sequence with a length in a range of 1 to 100 bp, 100 to 200 bp, 200 to 300 bp, 300 to 400 bp, 400 to 500 bp, 500 to 600 bp, 600 to 700 bp, 700 to 800 bp, or 800 to 900 bp. Preferably, the deleted nucleotide sequences at the 5' end of the MPZ promoter may be a nucleotide sequence with a length in a range of 800 to 900 bp. At this time, the artificially engineered rat MPZ promoter may have a sequence length in a range of 100 to 1000 bp, 100 to 900 bp, 100 to 800 bp, 100 to 700 bp, 100 to 600 bp, 100 to 500 bp, 100 to 400 bp, 100 to 300 bp, or 100 to 200 bp. Preferably, the artificially engineered rat MPZ promoter may have a sequence length in a range of 200 to 300 bp.

The Schwann cell-specific promoter may include a TATA box or CAAT box.

The Schwann cell-specific promoter may include a transcription start site (TSS).

The IE may include Egr2 and Sox10 binding site. The IE may be a region including the Egr2 and Sox10 binding site present in the intron 1 region of the MPZ gene. The IE may have a sequence length in a range of 1 to 100 bp, 100 to 200 bp, 200 to 300 bp, or 300 to 400 bp. Preferably, the IE may have a sequence length in a range of 100 to 200 bp, and more preferably, the IE may have a sequence length of 117 bp. In another embodiment, the IE may be the nucleotide sequence shown in SEQ ID NO: 7 with a length of 117 bp. Alternatively, the IE may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 7.

In another embodiment, the Schwann cell-specific promoter may be in a form where the IE, which has the nucleotide sequence shown in SEQ ID NO: 7 with a length of 117 bp, is connected to the 3' end of the artificially engineered rat MPZ promoter, which has the nucleotide sequence shown in SEQ ID NO: 3 with a length of 300 bp, and the Schwann cell-specific promoter may comprise a nucleotide sequence shown in SEQ ID NO: 13: 5'-GCCTCACCCCACCCCAACATTCCAACCTAGGGTAGGGGGAGGTCAGTATACACAAAGCCCTCT GTGTAAGGGGTGGTATGTGTCCCCCCACCCCCCTACCCAGAGTATACAATGCCCCTTCTGCTC CATGCCCCTGCCACCCTCCCCACCACCTCTCAATTGCACATGCCAGGCTGCAATTGGTCACTG GCTCAGGACAGCCCCCTCATGCTGGGGATCCAGGGGATTTTAAGCAGGTTCCAGAAAACACCA CTCAGTTCCTTGTCCCCCGCTCTCTCCACCCCACAGACGCTCTGGGCCGACAATGAGACTTTG TTTGGTCGCCTCCTCCTAAAGAACAGAAAAGTCTATAATTGTTCCTCCCCAGAGCCAGCCCAC ACACATAGACTGCCTGGGATGACTCTCCCTGTGTGGGCG-3' (SEQ ID NO: 13). At this time, the Schwann cell-specific promoter may have a sequence length of 417 bp. Alternatively, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 13.

In another embodiment, the Schwann cell-specific promoter may be in a form where the IE, which has the nucleotide sequence shown in SEQ ID NO: 7 with a length of 117 bp, is connected to the 3' end of the artificially engineered rat MPZ promoter, which has the nucleotide sequence shown in SEQ ID NO: 4 with a length of 200 bp, and the Schwann cell-specific promoter may comprise a nucleotide sequence shown in SEQ ID NO: 14: 5'-CAGAGTATACAATGCCCCTTCTGCTCCATGCCCCTGCCACCCTCCCCACCACCTCTCAATTGC ACATGCCAGGCTGCAATTGGTCACTGGCTCAGGACAGCCCCCTCATGCTGGGGATCCAGGGGA TTTTAAGCAGGTTCCAGAAAACACCACTCAGTTCCTTGTCCCCCGCTCTCTCCACCCCACAGA CGCTCTGGGCCGACAATGAGACTTTGTTTGGTCGCCTCCTCCTAAAGAACAGAAAAGTCTATA ATTGTTCCTCCCCAGAGCCAGCCCACACACATAGACTGCCTGGGATGACTCTCCCTGTGTGGG CG-3' (SEQ ID NO: 14) . At this time, the Schwann cell-specific promoter may have a sequence length of 317 bp. Alternatively, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 14.

In one embodiment, the Schwann cell-specific promoter comprises the artificially engineered human MPZ promoter in which some nucleotide sequences of the human MPZ promoter have been deleted and comprises the IE which is positioned at the 3' end of the artificially engineered human MPZ promoter.

The Schwann cell-specific promoter may be an artificially engineered human MPZ promoter in which some nucleotide sequences at the 5' end of the human MPZ promoter have been deleted. At this time, the deleted nucleotide sequences at the 5' end of the MPZ promoter may be a nucleotide sequence with a length in a range of 1 to 100 bp, 100 to 200 bp, 200 to 300 bp, 300 to 400 bp, 400 to 500 bp, 500 to 600 bp, 600 to 700 bp, 700 to 800 bp, or 800 to 900 bp. Preferably, the deleted nucleotide sequences at the 5' end of the MPZ promoter may be a nucleotide sequence with a length in a range of 800 to 900 bp. At this time, the artificially engineered human MPZ promoter may have a sequence length in a range of 100 to 1000 bp, 100 to 900 bp, 100 to 800 bp, 100 to 700 bp, 100 to 600 bp, 100 to 500 bp, 100 to 400 bp, 100 to 300 bp, or 100 to 200 bp. Preferably, the artificially engineered human MPZ promoter may have a sequence length in a range of 200 to 300 bp.

The Schwann cell-specific promoter may include a TATA box or CAAT box.

The Schwann cell-specific promoter may include a transcription start site (TSS).

The IE may include Egr2 and Sox10 binding site. The IE may be a region including the Egr2 and Sox10 binding site present in the intron 1 region of the MPZ gene. The IE may have a sequence length in a range of 1 to 100 bp, 100 to 200 bp, 200 to 300 bp, or 300 to 400 bp. Preferably, the IE may have a sequence length in a range of 100 to 200 bp, and more preferably, the IE may have a sequence length of 123 bp. In yet another embodiment, the IE may be the nucleotide sequence shown in SEQ ID NO: 10 with a length of 123 bp. Alternatively, the IE may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 10.

In another embodiment, the Schwann cell-specific promoter may be in a form where the IE, which has the nucleotide sequence shown in SEQ ID NO: 10 with a length of 123 bp, is connected to the 3' end of the artificially engineered human MPZ promoter, which has the nucleotide sequence shown in SEQ ID NO: 5 with a length of 300 bp, and the Schwann cell-specific promoter may comprise a nucleotide sequence shown in SEQ ID NO: 15: 5'- 
GTCCCTCTCCCTCACCACCCCCCACAACATTCCAGCCTGGGGCAGGGGGAGGCCAGTGGACAC

AAAGCCCTCTGTGTATGGGGTGGTATGTGTCCCCCCACCCCTCCACCCAGACTATACAATGCC

CCTTCTGCTCCCTGCACTCTGCCCCCCTCCCCACCACCTCTCAACTGCACATGCCAGGCTGCA

ATTGGTTACTGGCTGAGGACAGCCCCCTCATGCTGGGGCCCTAGGGGATTTTAAGCAGGTTCC

AGGAACCCCCCGTTCAGTTCCTGGTCCCCCACTTTCTCAACCCCACAGGACAATGGAACTTTG

TTTGGTTGCCACCTCCCCCTCCTGTAGAACAAAAAGGTCTACAGTTGCCCCTCCCTGGGGCCA

GCCCACACACATAGTCTCTCTGGAATGACCTTCCTTGTGTGGGTA-3′ (SEQ ID NO: 15).
 At this time, the Schwann cell-specific promoter may have a sequence length of 423 bp. Alternatively, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 15.

In another embodiment, the Schwann cell-specific promoter may be in a form where the IE, which has the nucleotide sequence shown in SEQ ID NO: 10 with a length of 123 bp, is connected to the 3' end of the artificially engineered human MPZ promoter, which has the nucleotide sequence shown in SEQ ID NO: 6 with a length of 200 bp, and the Schwann cell-specific promoter may consist of a nucleotide sequence shown in SEQ ID NO: 16: 5'-CCCCTCCACCCAGACTATACAATGCCCCTTCTGCTCCCTGCACTCTGCCCCCCTCCCCACCAC CTCTCAACTGCACATGCCAGGCTGCAATTGGTTACTGGCTGAGGACAGCCCCCTCATGCTGGG GCCCTAGGGGATTTTAAGCAGGTTCCAGGAACCCCCCGTTCAGTTCCTGGTCCCCCACTTTCT CAACCCCACAGGACAATGGAACTTTGTTTGGTTGCCACCTCCCCCTCCTGTAGAACAAAAAGG TCTACAGTTGCCCCTCCCTGGGGCCAGCCCACACACATAGTCTCTCTGGAATGACCTTCCTTG TGTGGGTA-3' (SEQ ID NO: 16). At this time, the Schwann cell-specific promoter may have a sequence length of 323 bp. Alternatively, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NO: 16.

### 1-4. Advantage 1 of Schwann cell-specific promoter - short length and high expression rate

The Schwann cell-specific promoter of the present disclosure has an advantage of being shorter in length than the known MPZ promoter. The advantage can be found in the fact that, when positioning the promoter within a vector to express a gene, a shorter promoter is more advantageous in terms of size compared to a longer promoter.

Additionally, the Schwann cell-specific promoter has an advantage of having a higher expression rate than the known MPZ promoter. The high expression rate of the Schwann cell-specific promoter was confirmed in the experiment of Experimental Example 1. In other words, the Schwann cell-specific promoter of the present disclosure exhibits a higher expression rate even though the Schwann cell-specific promoter has a length shorter than that of the known MPZ promoter.

In another embodiment, it is useful for a user to load all the configurations, that he or she wishes to introduce, into one vector. At this time, in generally, size constraints in the expression cassette in general (specifically in the promoter sequence) are particularly important. For example, the Schwann cell-specific promoter is advantageous over the larger MPZ promoter because of the relatively compact size of the promotor. This makes the Schwann cell-specific promoter more efficient when used in a limited-sized vector.

In yet another embodiment, the known MPZ promoter may be the promoter of the human or rat MPZ gene. A sequence having a length in a range of 1000 to 1200 bp is generally used for the known MPZ promoter.

In yet another embodiment, the Schwann cell-specific promoter having a sequence selected from SEQ ID NO: 3; SEQ ID NO: 4; SEQ ID NO: 5; SEQ ID NO: 6; SEQ ID NO: 8; SEQ ID NO: 9; SEQ ID NO: 11; SEQ ID NO: 12; SEQ ID NO: 13; SEQ ID NO: 14; SEQ ID NO: 15; SEQ ID NO: 16; and a sequence having at least 90% homology with one of SEQ ID NOs: 3, 4, 5, 6, 8, 9, 11, 12, 13, 14, 15, and 16 has a length in a range of 200 to 420 bp. The length of the Schwann cell-specific promoter may be 50% or less of the length of the known MPZ promoter. Specifically, when the Schwann cell-specific promoter has a length of 200bp, 300bp, or 317bp, the length may correspond to 30% or less of the length of the known MPZ promoter.

In yet another embodiment, the Schwann cell-specific promoter having a sequence selected from SEQ ID NO: 3; SEQ ID NO: 4; SEQ ID NO: 5; SEQ ID NO: 6; SEQ ID NO: 8; SEQ ID NO: 9; SEQ ID NO: 11; SEQ ID NO: 12; SEQ ID NO: 13; SEQ ID NO: 14; SEQ ID NO: 15; SEQ ID NO: 16; and a sequence having at least 90% homology with one of SEQ ID NOs: 3, 4, 5, 6, 8, 9, 11, 12, 13, 14, 15, and 16, despite being at least half the length of the known MPZ promoter, exhibits higher expression level compared to the known MPZ promoter. In addition, in yet another embodiment, through Experimental Examples 1-4, it was confirmed that the Schwann cell-specific promoter may achieve an approximately 1.5 to 2 times higher expression rate of the protein of interest than that achieved by the known MPZ promoter.

Therefore, in the case of size constraints in the vector, the Schwann cell-specific promoter having a sequence selected from SEQ ID NO: 3; SEQ ID NO: 4; SEQ ID NO: 5; SEQ ID NO: 6; SEQ ID NO: 8; SEQ ID NO: 9; SEQ ID NO: 11; SEQ ID NO: 12; SEQ ID NO: 13; SEQ ID NO: 14; SEQ ID NO: 15; SEQ ID NO: 16; and a sequence having at least 90% homology with one of SEQ ID NOs: 3, 4, 5, 6, 8, 9, 11, 12, 13, 14, 15, and 16 may be used more efficiently than the known MPZ promoter.

### 1-5. Advantage 2 of Schwann cell-specific promoter - specific expression in Schwann cells

The promoter of the present disclosure has the advantage (feature) of being able to specifically express a protein of interest in Schwann cells. That is, the Schwann cell-specific promoter is a promoter that causes significant expression in Schwann cells and low expression or no expression in non-Schwann cells.

In one embodiment, a vector for expressing a gene of interest in the body may be injected through systemic administration. At this time, the gene of interest needs to be expressed specifically for a specific type of tissue or cell.

When the gene of interest is expressed in tissues or cells other than those where the gene is specifically intended to be expressed, unpredictable side effects may probably increase, contrary to the desired effect sought through the gene expression. In other words, it is necessary to use a promoter that better selectively expresses the gene of interest in a specific tissue or cell.

For example, when a promoter is used to specifically express a gene of interest in Schwann cells, the Schwann cell-specific promoter of the present disclosure is more effective than a promoter non-specific to Schwann cells.

In another embodiment, a composition including a vector for expressing a gene of interest may be used as a gene therapy. In yet another embodiment of a method of treatment using gene therapy in vivo, there is a method of directly applying gene therapy to the subject's body to express a gene of interest in a specific tissue or cell. At this time, the vector travels through the blood in the body and passes through tissues such as the liver in addition to the tissue of interest. Therefore, for effective gene therapy, it is preferable for the gene therapy to exhibit low gene expression in tissues other than the tissue of interest (such as the liver) and to exhibit the most effective expression specifically in the tissue of interest. The promoter of the present disclosure is a Schwann cell-specific promoter that may effectively express a gene of interest in neural tissue.

In yet another embodiment, when using the promoter described below in the present disclosure to specifically express a gene of interest in Schwann cells, the protein expression rate in Schwann cells is higher than a known Schwann cell-specific promoter and the protein expression rate in non-Schwann cells is relatively lower than the known Schwann cell-specific promoter:

the Schwann cell-specific promoter having a sequence selected from SEQ ID NO: 3; SEQ ID NO: 4; SEQ ID NO: 5; SEQ ID NO: 6; SEQ ID NO: 8; SEQ ID NO: 9; SEQ ID NO: 11; SEQ ID NO: 12; SEQ ID NO: 13; SEQ ID NO: 14; SEQ ID NO: 15; SEQ ID NO: 16; or a sequence having at least 90% homology with one of SEQ ID NOs: 3, 4, 5, 6, 8, 9, 11, 12, 13, 14, 15, and 16.

However, the examples of Schwann cell-specific promoters disclosed above are merely examples and are not limited thereto.

### 2. Schwann cell-specific expression vector

In one aspect, the present disclosure relates to a Schwann cell-specific expression vector. The Schwann cell-specific expression vector includes a Schwann cell-specific promoter. The Schwann cell-specific promoter is the same as the promoter described above in section "1. Schwann cell-specific promoter" and has the same properties and structure as each configuration described in the section. The Schwann cell-specific expression vector refers to a vector which specifically expressed in Schwann cells. This means that the vector operates specifically in Schwann cells by the Schwann cell-specific promoter comprised in the Schwann cell-specific expression vector.

More specifically, the Schwann cell-specific expression vector may include a Schwann cell-specific promoter and a gene of interest.

The Schwann cell-specific promoter is the same as the promoter described above in section "1. Schwann cell-specific promoter" and has the same properties and structure as each configuration described in the section.

The gene of interest may be a gene configured to be expressed in Schwann cells. In addition, the gene of interest may be a nucleic acid encoding a protein of interest configured to be expressed in Schwann cells.

The Schwann cell-specific expression vector may selectively further include a regulatory/control element. At this time, the regulatory/control component may be operably linked to the gene of interest included in the vector. The regulatory/control component may include an enhancer, artificial intron, termination signal, polyadenylation signal, Kozak consensus sequence, internal ribosome entry site (IRES), WPRE, splice acceptor, 2A sequence, and/or replication origin but is not limited thereto.

The Schwann cell-specific expression vector may be a viral vector or non-viral vector. Herein, the viral vector may be one or more viral vectors selected from the group consisting of a retroviral (retrovirus) vector, lentiviral (lentivirus) vector, adenoviral (adenovirus) vector, adeno-associated viral (adeno-associated virus; AAV) vector, vaccinia viral (vaccinia virus) vector, poxviral (poxvirus) vector, and herpes simplex viral (herpes simplex virus) vector. At this time, the non-viral vector may be a plasmid, phage, naked DNA, DNA complex, mRNA (transcript), or PCR amplicon but is not limited thereto. For example, the plasmid may be any selected from the group consisting of pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19.

### 2-1. Example of Schwann cell-specific expression vector (1)

In one embodiment, the Schwann cell-specific expression vector may include a Schwann cell-specific promoter and a gene of interest. The gene of interest may be operably linked to the Schwann cell-specific promoter.

The Schwann cell-specific promoter may be an artificially engineered rat MPZ promoter in which some nucleotide sequences of the rat MPZ promoter have been deleted. In another embodiment, the Schwann cell-specific promoter may have a nucleotide sequence shown in SEQ ID NOs: 3 or 4. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 3 or 4.

The Schwann cell-specific promoter may comprise the artificially engineered rat MPZ promoter in which some nucleotide sequences of the rat MPZ promoter have been deleted and comprise the IE which is positioned at the 5' end of the artificially engineered rat MPZ promoter. In yet another embodiment, the Schwann cell-specific promoter may have a nucleotide sequence shown in SEQ ID NOs: 8 or 9. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 8 or 9.

The Schwann cell-specific promoter may comprise the artificially engineered rat MPZ promoter in which some nucleotide sequences of the rat MPZ promoter have been deleted and comprise the IE which is positioned at the 3' end of the artificially engineered rat MPZ promoter. In yet another embodiment, the Schwann cell-specific promoter may have a nucleotide sequence shown in SEQ ID NOs: 13 or 14. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 13 or 14.

The gene of interest may be a gene associated with diseases related to Schwann cells. For example, the gene associated with diseases related to Schwann cells may be a connexin 32 (Cx32) gene, SH3 domain and tetratricopeptide repeats 2 (SH3TC2) gene, myelin protein zero (MPZ) gene, early growth response 2 (EGR2) gene, ganglioside induced differentiation associated protein 1 (GDAP1) gene, N-Myc downstream regulated 1 (NDRG1) gene, or peripheral myelin protein 22 (PMP22) gene but is not limited thereto.

The gene of interest may be a nucleic acid encoding a protein for artificially engineering genes in Schwann cells. For example, the protein for artificially engineering genes may be Cas protein, zinc finger nuclease (ZFN), or tranion activator-like effector nuclease (TALEN) but is not limited thereto. Herein, the Cas protein may be Cas9 protein, Cas12a1 (Cpf1) protein, or Cas12f1 protein but are not limited thereto.

The Schwann cell-specific expression vector may be a viral vector. Herein, the viral vector may be one or more viral vectors selected from the group consisting of a retroviral (retrovirus) vector, lentiviral (lentivirus) vector, adenoviral (adenovirus) vector, adeno-associated viral (adeno-associated virus; AAV) vector, vaccinia viral (vaccinia virus) vector, poxviral (poxvirus) vector, and herpes simplex viral (herpes simplex virus) vector.

In yet another embodiment, the Schwann cell-specific expression vector may include a Schwann cell-specific promoter and a nucleic acid encoding the Cas9 protein. The nucleic acid encoding the Cas9 protein may be operably linked to the Schwann cell-specific promoter. At this time, the Schwann cell-specific promoter may have a nucleotide sequence shown in SEQ ID NOs: 3 or 4. At this time, the Cas9 protein may be a *Streptococcus pyogenes* Cas9 (SpCas9) protein, a *Staphylococcus aureus* Cas9 (SaCas9) protein, a *Campylobacter jejuni* Cas9 (CjCas9) protein, or a *Staphylococcus auricularis* Cas9 (SauriCas9) protein. At this time, the Schwann cell-specific expression vector may be an adeno-associated virus vector (AAV vector).

In yet another embodiment, the Schwann cell-specific expression vector may include a Schwann cell-specific promoter and a nucleic acid encoding the Cas9 protein. At this time, the nucleic acid encoding the Cas9 protein may be operably linked to the Schwann cell-specific promoter. At this time, the Schwann cell-specific promoter may have a nucleotide sequence shown in SEQ ID NOs: 8 or 9. Herein, the Cas9 protein may be SpCas9 protein, SaCas9 (CjCas9) protein, or SauriCas9 protein. At this time, the Schwann cell-specific expression vector may be an adeno-associated virus vector (AAV vector).

In yet another embodiment, the Schwann cell-specific expression vector may include a Schwann cell-specific promoter and a nucleic acid encoding the Cas9 protein. At this time, the nucleic acid encoding the Cas9 protein may be operably linked to the Schwann cell-specific promoter. At this time, the Schwann cell-specific promoter may have a nucleotide sequence shown in SEQ ID NOs: 13 or 14. Herein, the Cas9 proteins may be SpCas9 protein, SaCas9 (CjCas9) protein, or SauriCas9 protein. At this time, the Schwann cell-specific expression vector may be an adeno-associated virus vector (AAV vector).

### 2-2. Example of Schwann cell-specific expression vector (2)

In one embodiment, the Schwann cell-specific expression vector may include a Schwann cell-specific promoter and a gene of interest. The gene of interest may be operably linked to the Schwann cell-specific promoter.

The Schwann cell-specific promoter may be an artificially engineered human MPZ promoter in which some nucleotide sequences of the human MPZ promoter have been deleted. In another embodiment, the Schwann cell-specific promoter may have a nucleotide sequence shown in SEQ ID NOs: 5 or 6. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 5 or 6.

The Schwann cell-specific promoter may comprise the artificially engineered human MPZ promoter in which some nucleotide sequences of the human MPZ promoter have been deleted and comprise the IE which is positioned at the 5' end of the artificially engineered human MPZ promoter. In yet another embodiment, the Schwann cell-specific promoter may have a nucleotide sequence shown in SEQ ID NOs: 11 or 12. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 11 or 12.

The Schwann cell-specific promoter may comprise the artificially engineered human MPZ promoter in which some nucleotide sequences of the human MPZ promoter have been deleted and comprise the IE which is positioned at the 3' end of the artificially engineered human MPZ promoter. In yet another embodiment, the Schwann cell-specific promoter may have a nucleotide sequence shown in SEQ ID NOs: 15 or 16. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 15 or 16.

The gene of interest may be a gene associated with diseases related to Schwann cells. For example, the gene associated with diseases related to Schwann cells may be Cx32 gene, SH3TC2 gene, MPZ gene, EGR2 gene, GDAP1 gene, NDRG1 gene, or PMP22 gene but is not limited thereto.

The gene of interest may be a nucleic acid encoding a protein for artificially engineering genes in Schwann cells. For example, the protein used for artificially engineering genes may be Gas protein, ZFN, or TALEN but is not limited thereto. Herein, the Gas protein may be Cas9 protein, Cas12a1 (Cpf1) protein, or Cas12f1 protein but are not limited thereto.

The Schwann cell-specific expression vector may be a viral vector. At this time, the viral vector may be one or more selected from the group consisting of a retroviral vector, lentiviral vector, adenovirus vector, adeno-associated virus vector, vaccinia virus vector, poxvirus vector, and herpes simplex virus vector.

In yet another embodiment, the Schwann cell-specific expression vector may include a Schwann cell-specific promoter and a nucleic acid encoding the Cas9 protein. At this time, the nucleic acid encoding the Cas9 protein may be operably linked to the Schwann cell-specific promoter. At this time, the Schwann cell-specific promoter may have a nucleotide sequence shown in SEQ ID NOs: 5 or 6. Herein, the Cas9 proteins may be SpCas9 protein, SaCas9 protein, CjCas9 protein, or SauriCas9 protein. At this time, the Schwann cell-specific expression vector may be an adeno-associated virus vector (AAV vector).

In yet another embodiment, the Schwann cell-specific expression vector may include a Schwann cell-specific promoter and a nucleic acid encoding the Cas9 protein. At this time, the nucleic acid encoding the Cas9 protein may be operably linked to the Schwann cell-specific promoter. At this time, the Schwann cell-specific promoter may have a nucleotide sequence shown in SEQ ID NOs: 11 or 12. Herein, the Cas9 proteins may be SpCas9 protein, SaCas9 protein, CjCas9 protein, or SauriCas9 protein. At this time, the Schwann cell-specific expression vector may be an adeno-associated virus vector (AAV vector).

In yet another embodiment, the Schwann cell-specific expression vector may include a Schwann cell-specific promoter and a nucleic acid encoding the Cas9 protein. At this time, the nucleic acid encoding the Cas9 protein may be operably linked to the Schwann cell-specific promoter. At this time, the Schwann cell-specific promoter may have a nucleotide sequence shown in SEQ ID NOs: 15 or 16. Herein, the Cas9 proteins may be SpCas9 protein, SaCas9 protein, CjCas9 protein, or SauriCas9 protein. At this time, the Schwann cell-specific expression vector may be an adeno-associated virus vector (AAV vector).

### 2-3. Example of Schwann cell-specific expression vector (3)

In one embodiment, the Schwann cell-specific expression vector may include a Schwann cell-specific promoter, gene of interest, and regulatory/control element. At this time, the gene of interest may be operably linked to the Schwann cell-specific promoter.

The Schwann cell-specific promoter may be an artificially engineered rat MPZ promoter in which some nucleotide sequences of the rat MPZ promoter have been deleted. In another embodiment, the Schwann cell-specific promoter may have a nucleotide sequence shown in SEQ ID NOs: 3 or 4. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 3 or 4.

The Schwann cell-specific promoter may comprise the artificially engineered rat MPZ promoter in which some nucleotide sequences of the rat MPZ promoter have been deleted and comprise the IE which is positioned at the 5' end of the artificially engineered rat MPZ promoter. In yet another embodiment, the Schwann cell-specific promoter may have a nucleotide sequence shown in SEQ ID NOs: 8 or 9. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 8 or 9.

The Schwann cell-specific promoter may comprise the artificially engineered rat MPZ promoter in which some nucleotide sequences of the rat MPZ promoter have been deleted and comprise the IE which is positioned at the 3' end of the artificially engineered rat MPZ promoter. In yet another embodiment, the Schwann cell-specific promoter may have a nucleotide sequence shown in SEQ ID NOs: 13 or 14. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 13 or 14.

The gene of interest may be a gene associated with diseases related to Schwann cells. For example, the gene associated with diseases related to Schwann cells may be Cx32 gene, SH3TC2 gene, MPZ gene, EGR2 gene, GDAP1 gene, NDRG1 gene, or PMP22 gene but is not limited thereto.

The gene of interest may be a nucleic acid encoding a protein for artificially engineering genes in Schwann cells. For example, the protein used for artificially engineering genes may be Gas protein, ZFN, or TALEN but is not limited thereto. Herein, the Gas protein may be Cas9 protein, Cas12a1 (Cpf1) protein, or Cas12f1 protein but are not limited thereto.

The regulatory/control element may be a polyadenylation signal, a Woodchuck hepatitis virus posttranscriptional regulatory element (WPRE), and/or an artificial intron.

At this time, the polyadenylation signal may be a bovine growth hormone polyadenylation signal (BGHA), synthetic polyadenylation signal (synpA), or simian virus 40 polyadenylation signal (SV40pA) but is not limited thereto. In yet another embodiment, the SV40pA may have a nucleotide sequence shown in SEQ ID NO: 17: 5'-tgctttatttgtaaccattataagctgcaataaacaagttaacaacaacaattgcattcattt tatgtttcaggttcagggggagatgtgggaggttttttaaa-3' (SEQ ID NO: 17). In yet another embodiment, SV40pA may have a nucleotide sequence shown in SEQ ID NO: 18: 5'-AAGAGGTAAGGGTTTAAGGGATGGTTGGTTGGTGGGGTATTAATGTTTAATTACCTGGAGCAC CTGCCTGAAATCACTTTTTTTCAGGTTGG-3' (SEQ ID NO: 18).

At this time, the WPRE may have one or more elements selected from gamma, alpha, and beta elements. In yet another embodiment, the WPRE may be WPRE3 including gamma and alpha elements, and the WPRE3 may have a nucleotide sequence shown in SEQ ID NO: 19: 5'-AATCAACCTCTGGATTACAAAATTTGTGAAAGATTGACTGGTATTCTTAACTATGTTGCTCCT TTTACGCTATGTGGATACGCTGCTTTAATGCCTTTGTATCATGCTATTGCTTCCCGTATGGCT TTCATTTTCTCCTCCTTGTATAAATCCTGGTTAGTTCTTGCCACGGCGGAACTCATCGCCGCC TGCCTTGCCCGCTGCTGGACAGGGGCTCGGCTGTTGGGCACTGACAATTCCGTGGTGT-3' (SEQ ID NO: 19). In yet another embodiment, the WPRE may include gamma, alpha and beta elements. At this time, the WPRE may have a nucleotide sequence shown in SEQ ID NO: 20: 5'-AATCAACCTCTGGATTACAAAATTTGTGAAAGATTGACTGGTATTCTTAACTATGTTGCTCCT TTTACGCTATGTGGATACGCTGCTTTAATGCCTTTGTATCATGCTATTGCTTCCCGTATGGCT TTCATTTTCTCCTCCTTGTATAAATCCTGGTTGCTGTCTCTTTATGAGGAGTTGTGGCCCGTT GTCAGGCAACGTGGCGTGGTGTGCACTGTGTTTGCTGACGCAACCCCCACTGGTTGGGGCATT GCCACCACCTGTCAGCTCCTTTCCGGGACTTTCGCTTTCCCCCTCCCTATTGCCACGGCGGAA CTCATCGCCGCCTGCCTTGCCCGCTGCTGGACAGGGGCTCGGCTGTTGGGCACTGACAATTCC GTGGTGTTGTCGGGGAAGCTGACGTCCTTTCCATGGCTGCTCGCCTGTGTTGCCACCTGGATT CTGCGCGGGACGTCCTTCTGCTACGTCCCTTCGGCCCTCAATCCAGCGGACCTTCCTTCCCGC GGCCTGCTGCCGGCTCTGCGGCCTCTTCCGCGTCTTCGCCTTCGCCCTCAGACGAGTCGGATC TCCCTTTGGGCCGCCTCCCCGCCTG-3' (SEQ ID NO: 20).

At this time, the artificial intron may be used to improve the expression of the gene of interest in the Schwann cell-specific expression vector. In yet another embodiment, the artificial intron may be a minute virus of mice (MVM) intron. At this time, the MVM intron may have a nucleotide sequence shown in SEQ ID NO: 21: 5'-AAGAGGTAAGGGTTTAAGGGATGGTTGGTTGGTGGGGTATTAATGTTTAATTACCTGGAGCAC CTGCCTGAAATCACTTTTTTTCAGGTTGG-3' (SEQ ID NO: 21).

The Schwann cell-specific expression vector may be a viral vector. At this time, the viral vector may be one or more virus vectors selected from the group consisting of a retroviral vector, lentiviral vector, adenovirus vector, adeno-associated virus vector, vaccinia virus vector, poxvirus vector, and herpes simplex virus vector.

In yet another embodiment, the Schwann cell-specific expression vector may include a Schwann cell-specific promoter, nucleic acid encoding the Cas9 protein, and polyadenylation signal. At this time, the nucleic acid encoding the Cas9 protein may be operably linked to the Schwann cell-specific promoter. At this time, the Schwann cell-specific promoter may have a nucleotide sequence shown in SEQ ID NOs: 3, 4, 8, 9, 13, or 14. Herein, the Cas9 protein may be SpCas9 protein, SaCas9 protein, CjCas9 protein, or SauriCas9 protein. Herein, the polyadenylation signal may be a synpA or SV40pA. In yet another embodiment, the SV40pA may have a nucleotide sequence shown in SEQ ID NO: 17. In yet another embodiment, the synp A may have a nucleotide sequence shown in SEQ ID NO: 18. At this time, the polyadenylation signal may be located at the 3' end of the nucleic acid encoding the Cas9 protein. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, and WPRE. At this time, the nucleic acid encoding the Cas9 protein may be operably linked to the Schwann cell-specific promoter. At this time, the Schwann cell-specific promoter may have a nucleotide sequence shown in SEQ ID NOs: 3, 4, 8, 9, 13, or 14. Herein, the Cas9 protein may be SpCas9 protein, SaCas9 protein, CjCas9 protein, or SauriCas9 protein. At this time, the WPRE may be WPRE3. At this time, the WPRE3 may have a nucleotide sequence shown in SEQ ID NO: 19. At this time, the WPRE may be located at the 3' end of the nucleic acid encoding the Cas9 protein. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, and artificial intron. At this time, the nucleic acid encoding the Cas9 protein may be operably linked to the Schwann cell-specific promoter. At this time, the Schwann cell-specific promoter may have a nucleotide sequence shown in SEQ ID NOs: 3, 4, 8, 9, 13, or 14. Herein, the Cas9 protein may be SpCas9 protein, SaCas9 protein, CjCas9 protein, or SauriCas9 protein. At this time, the artificial intron may be an MVM intron. At this time, the MVM intron may have a nucleotide sequence shown in SEQ ID NO: 21. At this time, the MVM intron may be located between the Schwann cell-specific promoter and the nucleic acid encoding the Cas9 protein. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, WPRE, and polyadenylation signal. At this time, the nucleic acid encoding the Cas9 protein may be operably linked to the Schwann cell-specific promoter. At this time, the Schwann cell-specific promoter may have a nucleotide sequence shown in SEQ ID NOs: 3, 4, 8, 9, 13, or 14. Herein, the Cas9 protein may be SpCas9 protein, SaCas9 protein, CjCas9 protein, or SauriCas9 protein. Herein, the polyadenylation signal may be a synpA or SV40pA. In yet another embodiment, the SV40pA may have a nucleotide sequence shown in SEQ ID NO: 17. In yet another embodiment, the synpA may have a nucleotide sequence shown in SEQ ID NO: 18. At this time, the polyadenylation signal may be located at the 3' end of the nucleic acid encoding the Cas9 protein. At this time, the WPRE may be WPRE3. At this time, the WPRE3 may have a nucleotide sequence shown in SEQ ID NO: 19. At this time, the WPRE may be located between the nucleic acid encoding the Cas9 protein and the polyadenylation signal. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, artificial intron, and polyadenylation signal. At this time, the nucleic acid encoding the Cas9 protein may be operably linked to the Schwann cell-specific promoter. At this time, the Schwann cell-specific promoter may have a nucleotide sequence shown in SEQ ID NOs: 3, 4, 8, 9, 13, or 14. Herein, the Cas9 protein may be SpCas9 protein, SaCas9 protein, CjCas9 protein, or SauriCas9 protein. Herein, the polyadenylation signal may be a synpA or SV40pA. At this time, the SV40pA may have a nucleotide sequence shown in SEQ ID NO: 17. In yet another embodiment, the synpA may have a nucleotide sequence shown in SEQ ID NO: 18. At this time, the polyadenylation signal may be located at the 3' end of the nucleic acid encoding the Cas9 protein. At this time, the MVM intron may have a nucleotide sequence shown in SEQ ID NO: 21. At this time, the MVM intron may be located between the Schwann cell-specific promoter and the nucleic acid encoding the Cas9 protein. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, artificial intron, and WPRE. At this time, the nucleic acid encoding the Cas9 protein may be operably linked to the Schwann cell-specific promoter. At this time, the Schwann cell-specific promoter may have a nucleotide sequence shown in SEQ ID NOs: 3, 4, 8, 9, 13, or 14. Herein, the Cas9 protein may be SpCas9 protein, SaCas9 protein, CjCas9 protein, or SauriCas9 protein. At this time, the MVM intron may have a nucleotide sequence shown in SEQ ID NO: 21. At this time, the MVM intron may be located between the Schwann cell-specific promoter and the nucleic acid encoding the Cas9 protein. At this time, the WPRE may be WPRE3. At this time, the WPRE3 may have a nucleotide sequence shown in SEQ ID NO: 19. At this time, the WPRE may be located at the 3' end of the nucleic acid encoding the Cas9 protein. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, artificial intron, WPRE, and polyadenylation signal. At this time, the nucleic acid encoding the Cas9 proteins may be operably linked to the Schwann cell-specific promoter. At this time, the Schwann cell-specific promoter may have a nucleotide sequence shown in SEQ ID NOs: 3, 4, 8, 9, 13, or 14. Herein, the Cas9 protein may be SpCas9 protein, SaCas9 protein, CjCas9 protein, or SauriCas9 protein. At this time, the MVM intron may have a nucleotide sequence shown in SEQ ID NO: 21. At this time, the MVM intron may be located between the Schwann cell-specific promoter and the nucleic acid encoding the Cas9 protein. At this time, the WPRE may be WPRE3. At this time, the WPRE3 may have a nucleotide sequence shown in SEQ ID NO: 19. At this time, the WPRE may be located at the 3' end of the nucleic acid encoding the Cas9 protein. Herein, the polyadenylation signal may be a synpA or SV40pA. At this time, the SV40pA may have a nucleotide sequence shown in SEQ ID NO: 17. In yet another embodiment, the synpA may have a nucleotide sequence shown in SEQ ID NO: 18. At this time, the polyadenylation signal may be located at the 3' end of the WPRE. At this time, the Schwann cell-specific expression vector may be an adeno-associated virus vector (AAV vector).

### 2-4. Example of Schwann cell-specific expression vector (4)

In one embodiment, the Schwann cell-specific expression vector may include a Schwann cell-specific promoter, gene of interest, and regulatory/control element. At this time, the gene of interest may be operably linked to the Schwann cell-specific promoter.

The Schwann cell-specific promoter may be an artificially engineered human MPZ promoter in which some nucleotide sequences of the human MPZ promoter have been deleted. In another embodiment, the Schwann cell-specific promoter may have a nucleotide sequence shown in SEQ ID NOs: 5 or 6. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 5 or 6.

The Schwann cell-specific promoter may comprise the artificially engineered human MPZ promoter in which some nucleotide sequences of the human MPZ promoter have been deleted and comprise the IE which is positioned at the 5' end of the artificially engineered human MPZ promoter. In yet another embodiment, the Schwann cell-specific promoter may have a nucleotide sequence shown in SEQ ID NOs: 11 or 12. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 11 or 12.

The Schwann cell-specific promoter may comprise the artificially engineered human MPZ promoter in which some nucleotide sequences of the human MPZ promoter have been deleted and comprise the IE which is positioned at the 3' end of the artificially engineered human MPZ promoter. In yet another embodiment, the Schwann cell-specific promoter may have a nucleotide sequence shown in SEQ ID NOs: 15 or 16. In yet another embodiment, the Schwann cell-specific promoter may have a nucleotide sequence having at least 70% or more, such as 70% to 75%, 75% to 80%, 80% to 85%, 85% to 90%, 90% to 95%, or 95% to 100% sequence identity or sequence similarity with the nucleotide sequence shown in SEQ ID NOs: 15 or 16.

The gene of interest may be a gene associated with diseases related to Schwann cells. For example, the gene associated with diseases related to Schwann cells may be Cx32 gene, SH3TC2 gene, MPZ gene, EGR2 gene, GDAP1 gene, NDRG1 gene, or PMP22 gene but is not limited thereto.

The gene of interest may be a nucleic acid encoding a protein used for artificially engineering genes in Schwann cells. For example, the protein used for artificially engineering genes may be Gas protein, ZFN, or TALEN but is not limited thereto. Herein, the Gas protein may be Cas9 protein, Cas12a1 (Cpf1) protein, or Cas12f1 protein but are not limited thereto.

The regulatory/control element may be a polyadenylation signal, WPRE, and/or artificial intron.

At this time, the polyadenylation signal may be a bovine growth hormone polyadenylation signal, synthetic polyadenylation signal, or a simian virus 40 polyadenylation signal but is not limited thereto. At this time, the SV40pA may have a nucleotide sequence shown in SEQ ID NO: 17. In yet another embodiment, the synpA may have a nucleotide sequence shown in SEQ ID NO: 18.

At this time, the WPRE may have one or more elements selected from gamma, alpha, and beta elements. In yet another embodiment, the WPRE may be WPRE3 including gamma and alpha elements, and the WPRE3 may have nucleotide sequence shown in SEQ ID NO: 19. In yet another embodiment, the WPRE may include gamma, alpha, and beta elements. At this time, the WPRE may have a nucleotide sequence shown in SEQ ID NO: 20.

At this time, the artificial intron may be used to improve the expression of the gene of interest in the Schwann cell-specific expression vector. In yet another embodiment, the artificial intron may be a minute virus of mice (MVM) intron. At this time, the MVM intron may have a nucleotide sequence shown in SEQ ID NO: 21.

The Schwann cell-specific expression vector may be a viral vector. At this time, the viral vector may be one or more vectors selected from the group consisting of a retroviral vector, lentiviral vector, adenovirus vector, adeno-associated virus vector, vaccinia virus vector, poxvirus vector, and herpes simplex virus vector.

In yet another embodiment, the Schwann cell-specific expression vector may include a Schwann cell-specific promoter, nucleic acid encoding the Cas9 protein, and polyadenylation signal. At this time, the nucleic acid encoding the Cas9 protein may be operably linked to the Schwann cell-specific promoter. At this time, the Schwann cell-specific promoter may have a nucleotide sequence shown in SEQ ID NOs: 5, 6, 11, 12, 15, or 16. Herein, the Cas9 protein may be SpCas9 protein, SaCas9 protein, CjCas9 protein, or SauriCas9 protein. Herein, the polyadenylation signal may be a synpA or SV40pA. In yet another embodiment, the SV40pA may have a nucleotide sequence shown in SEQ ID NO: 17. In yet another embodiment, the synpA may have a nucleotide sequence shown in SEQ ID NO: 18. At this time, the polyadenylation signal may be located at the 3' end of the nucleic acid encoding the Cas9 protein. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, and WPRE. At this time, the nucleic acid encoding the Cas9 protein may be operably linked to the Schwann cell-specific promoter. At this time, the Schwann cell-specific promoter may have a nucleotide sequence shown in SEQ ID NOs: 5, 6, 11, 12, 15, or 16. Herein, the Cas9 protein may be SpCas9 protein, SaCas9 protein, CjCas9 protein, or SauriCas9 protein. At this time, the WPRE may be WPRE3. At this time, the WPRE3 may have a nucleotide sequence shown in SEQ ID NO: 19. At this time, the WPRE may be located at the 3' end of the nucleic acid encoding the Cas9 protein. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, and artificial intron. At this time, the nucleic acid encoding the Cas9 protein may be operably linked to the Schwann cell-specific promoter. At this time, the Schwann cell-specific promoter may have a nucleotide sequence shown in SEQ ID NOs: 5, 6, 11, 12, 15, or 16. Herein, the Cas9 protein may be SpCas9 protein, SaCas9 protein, CjCas9 protein, or SauriCas9 protein. At this time, the artificial intron may be an MVM intron. At this time, the MVM may have a nucleotide sequence shown in SEQ ID NO: 21. At this time, the MVM intron may be located between the Schwann cell-specific promoter and the nucleic acid encoding the Cas9 protein. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, WPRE, and polyadenylation signal. At this time, the nucleic acid encoding the Cas9 protein may be operably linked to the Schwann cell-specific promoter. At this time, the Schwann cell-specific promoter may have a nucleotide sequence shown in SEQ ID NOs: 5, 6, 11, 12, 15, or 16. Herein, the Cas9 protein may include SpCas9 protein, SaCas9 protein, CjCas9 protein, or SauriCas9 protein. Herein, the polyadenylation signal may include a synpA or SV40pA. At this time, the SV40pA may have a nucleotide sequence shown in SEQ ID NO: 17. In yet another embodiment, the synpA may have a nucleotide sequence shown in SEQ ID NO: 18. At this time, the polyadenylation signal may be located at the 3' end of the nucleic acid encoding the Cas9 protein. At this time, the WPRE may be WPRE3. At this time, the WPRE3 may have a nucleotide sequence shown in SEQ ID NO: 19. At this time, the WPRE may be located between the nucleic acid encoding the Cas9 protein and the polyadenylation signal. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, artificial intron, and polyadenylation signal. At this time, the nucleic acid encoding the Cas9 protein may be operably linked to the Schwann cell-specific promoter. At this time, the Schwann cell-specific promoter may have a nucleotide sequence shown in SEQ ID NOs: 5, 6, 11, 12, 15, or 16. Herein, the Cas9 protein may include SpCas9 protein, SaCas9 protein, CjCas9 protein, or SauriCas9 protein. Herein, the polyadenylation signal may be a synpA or SV40pA. At this time, the SV40pA may have a nucleotide sequence shown in SEQ ID NO: 17. In yet another embodiment, the synpA may have a nucleotide sequence shown in SEQ ID NO: 18. At this time, the polyadenylation signal may be located at the 3' end of the nucleic acid encoding the Cas9 protein. At this time MVM intron may have a nucleotide sequence shown in SEQ ID NO: 21. At this time, the MVM intron may be located between the Schwann cell-specific promoter and the nucleic acid encoding the Cas9 proteins At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, artificial intron, and WPRE. At this time, the nucleic acid encoding the Cas9 protein may be operably linked to the Schwann cell-specific promoter. At this time, the Schwann cell-specific promoter may have a nucleotide sequence shown in SEQ ID NOs: 5, 6, 11, 12, 15, or 16. Herein, the Cas9 protein may be SpCas9 protein, SaCas9 protein, CjCas9 protein, or SauriCas9 protein. At this time MVM intron may have a nucleotide sequence shown in SEQ ID NO: 21. At this time, the MVM intron may be located between the Schwann cell-specific promoter and the nucleic acid encoding the Cas9 protein. At this time, the WPRE may be WPRE3. At this time, the WPRE3 may have a nucleotide sequence shown in SEQ ID NO: 19. At this time, the WPRE may be located at the 3' end of the nucleic acid encoding the Cas9 protein. At this time, the Schwann cell-specific expression vector may be an adeno-associated virus vector (AAV vector).

In yet another embodiment, the Schwann cell-specific expression vector may include a Schwann cell-specific promoter, nucleic acid encoding the Cas9 protein, artificial intron, WPRE, and polyadenylation signal. At this time, the nucleic acid encoding the Cas9 protein may be operably linked to the Schwann cell-specific promoter. At this time, the Schwann cell-specific promoter may have a nucleotide sequence shown in SEQ ID NOs: 5, 6, 11, 12, 15, or 16. Herein, the Cas9 protein may include SpCas9 protein, SaCas9 protein, CjCas9 protein, or SauriCas9 protein. At this time MVM intron may have a nucleotide sequence shown in SEQ ID NO: 21. At this time, the MVM intron may be located between the Schwann cell-specific promoter and the nucleic acid encoding the Cas9 protein. At this time, the WPRE may be WPRE3. At this time, the WPRE3 may have a nucleotide sequence shown in SEQ ID NO: 19. At this time, the WPRE may be located at the 3' end of the nucleic acid encoding the Cas9 protein. Herein, the polyadenylation signal may be a synpA or SV40pA. At this time, the SV40pA may have a nucleotide sequence shown in SEQ ID NO: 17. In yet another embodiment, the synpA may have a nucleotide sequence shown in SEQ ID NO: 18. At this time, the polyadenylation signal may be located at the 3' end of the WPRE. At this time, the Schwann cell-specific expression vector may be an adeno-associated virus vector (AAV vector).

However, the examples of the Schwann cell-specific expression vector disclosed above are merely examples and are not limited thereto.

### 3. Method for Schwann cell-specific expression of protein of interest

One aspect disclosed by the present disclosure relates to a method of expressing a protein of interest in Schwann cells. The method uses a Schwann cell-specific expression vector. More specifically, the method is a method of introducing (or transferring) a Schwann cell-specific expression vector including a nucleic acid (or a gene of interest) encoding the protein of interest into Schwann cells to express a protein of interest in Schwann cells. At this time, the Schwann cells may be human Schwann cells or non-human animal Schwann cells. The method can be performed in vitro, ex vivo, or in vivo. At this time, in vivo may refer to an environment of a human body including Schwann cells or the body of a non-human animal. The Schwann cell-specific expression vector is the same as the expression vector described above in section "2. Schwann cell-specific expression vector" and has the same properties and structure as each configuration described in the section.

### 3-1. Method example of specifically expressing protein of interest in Schwann cells (1)

In one embodiment, the method may be a method of expressing the protein of interest in Schwann cells. At this time, the method may involve treating or introducing a Schwann cell-specific expression vector into Schwann cells.

In another embodiment, the Schwann cell-specific expression vector may include a Schwann cell-specific promoter and nucleic acid (or gene of interest) encoding the protein of interest. At this time, the gene of interest may be operably linked to the Schwann cell-specific promoter.

The Schwann cell-specific expression vector is the same as the expression vector described above in section "2. Schwann cell-specific expression vector" and has the same properties and structure as configurations described in the section. In addition, the Schwann cell-specific promoter is the same as the promoter described above in section "1. Schwann cell-specific promoter" and has the same properties and structure as configurations described in the section. In addition, the gene of interest is the same as the gene described above in section "2. Schwann cell-specific expression vector" and has the same properties and structure as configurations described in the section.

The Schwann cells may be human Schwann cells or non-human animal Schwann cells.

The Schwann cells may be Schwann cells obtained from human or non-human animals.

The Schwann cells may be Schwann cells existing within human body or non-human animal body.

The treatment or introduction of the Schwann cell-specific expression vector into the Schwann cells may be performed using an electroporation method, gene gun, sonoporation, magnetofection method, nanoparticle method, and/or temporary cell compression or squeezing method. In addition, the treatment or introduction of the Schwann cell-specific expression vector into the Schwann cells may be performed using a cationic liposome method, lithium acetate-DMSO, lipid-mediated transfection, calcium phosphate precipitation, lipofection, polyethyleneimine (PEI)-mediated transfection, DEAE-dextran-mediated transfection, and/or nanoparticle-mediated nucleic acid delivery (see Panyam et al., Adv Drug Deliv Rev. 2012 Sep 13. pii: S0169-409X(12)00283-9. doi: 10.1016/j.addr.2012.09.023) but is not limited thereto.

The treatment or introduction of the Schwann cell-specific expression vector into the Schwann cells may be performed in vitro, ex vivo, or in vivo.

As a result of treating or introducing the Schwann cell-specific expression vector into the Schwann cells, the protein of interest may be expressed in the Schwann cells. At this time, the expression of the protein of interest in the Schwann cells may significantly increase compared to before treatment with the Schwann cell-specific expression vector.

In yet another embodiment, the method may be a method of expressing the protein of interest in Schwann cells. At this time, the method may involve processing or introducing a Schwann cell-specific expression vector into Schwann cells. The Schwann cell-specific expression vector may include a Schwann cell-specific promoter and a nucleic acid encoding the Cas9 protein. At this time, the nucleic acid encoding the Cas9 protein may be operably linked to the Schwann cell-specific promoter. The Schwann cell-specific expression vector may be one selected in several embodiments described above in sections "2-1. Example of Schwann cell-specific expression vector (1)" and "2-2. Example of Schwann cell-specific expression vector (2)". At this time, the Schwann cells may be human Schwann cells. At this time, the method may be performed in vitro, ex vivo, or in vivo. In addition, the method may further comprise treating or introducing a guide RNA or a nucleic acid encoding the guide RNA into Schwann cells.

### 3-2. Method example of specifically expressing protein of interest in Schwann cells (2)

In one embodiment, the method may be a method of expressing a protein of interest in Schwann cells. At this time, the method may comprise processing or introducing a Schwann cell-specific expression vector into Schwann cells.

At this time, the Schwann cell-specific expression vector may include a Schwann cell-specific promoter, nucleic acid (or gene of interest) encoding the protein of interest, and regulatory/control element. At this time, the gene of interest may be operably linked to the Schwann cell-specific promoter.

The Schwann cell-specific expression vector is the same as the expression vector described above in section "2. Schwann cell-specific expression vector" and has the same properties and structure as configurations described in the section. In addition, the Schwann cell-specific promoter is the same as the promoter described above in section "1. Schwann cell-specific promoter" and has the same properties and structure as configurations described in the section. In addition, the gene of interest and regulatory/control element are the same as the gene and regulatory/control element described above in section "2. Schwann cell-specific expression vector" and have the same properties and structure as configurations described in the section.

The Schwann cells may be human Schwann cells or non-human animal Schwann cells.

The Schwann cells may be Schwann cells obtained from human or non-human animals.

The Schwann cells may be Schwann cells present in human's body or non-human animal's body.

The treatment or introduction of the Schwann cell-specific expression vector into the Schwann cells may be performed using an electroporation method, gene gun, sonoporation, magnetofection method, nanoparticle method, and/or temporary cell compression or squeezing method. In addition, the treatment or introduction of the Schwann cell-specific expression vector into the Schwann cells may be performed using a cationic liposome method, lithium acetate-DMSO, lipid-mediated transfection, calcium phosphate precipitation, lipofection, polyethyleneimine (PEI)-mediated transfection, DEAE-dextran-mediated transfection, and/or nanoparticle-mediated nucleic acid delivery (see Panyam et al., Adv Drug Deliv Rev. 2012 Sep 13. pii: S0169-409X(12)00283-9. doi: 10.1016/j.addr.2012.09.023) but is not limited thereto.

The treatment or introduction of the Schwann cell-specific expression vector into the Schwann cells may be performed in vitro, ex vivo, or in vivo.

As a result of treating or introducing the Schwann cell-specific expression vector into the Schwann cells, the protein of interest may be expressed in the Schwann cells. At this time, the expression of the protein of interest in the Schwann cells may significantly increase compared to before treatment with the Schwann cell-specific expression vector.

In yet another embodiment, the method may be a method of expressing a protein of interest in Schwann cells. At this time, the method may comprise processing or introducing a Schwann cell-specific expression vector into Schwann cells. The Schwann cell-specific expression vector may include a Schwann cell-specific promoter, nucleic acid encoding the Cas9 protein, and regulatory/control element. At this time, the nucleic acid encoding the Cas9 protein may be operably linked to the Schwann cell-specific promoter. The Schwann cell-specific expression vector may be one selected in several embodiments described above in sections "2-3. Example of Schwann cell-specific expression vector (3)" and "2-4. Example of Schwann cell-specific expression vector (4)". At this time, the Schwann cells may be human Schwann cells. At this time, the method may be performed in vitro, ex vivo, or in vivo. In addition, the method may further comprise processing or introducing a guide RNA or a nucleic acid encoding the guide RNA into Schwann cells.

However, the examples of the method for Schwann cell-specific expression of the protein of interest disclosed above are merely examples and are not limited thereto.

### 4. Uses in treating diseases related to Schwann cells

### 4-1. Composition

One aspect disclosed by the present disclosure relates to a composition for treating diseases related to Schwann cells. The composition includes a Schwann cell-specific expression vector. The Schwann cell-specific expression vector is the same as the expression vector described above in section "2. Schwann cell-specific expression vector" and has the same properties and structure as configurations described in the section. The Schwann cell-specific expression vector may include a gene associated with diseases related to Schwann cells and/or nucleic acid encoding a protein for artificially engineering genes in Schwann cells as a gene of interest. Therefore, the composition may be used to treat diseases related to Schwann cells by using the gene associated with diseases related to Schwann cells and/or nucleic acid encoding the protein for artificially engineering genes in Schwann cells.

More specifically, the composition may include the Schwann cell-specific expression vector including the following:
Schwann cell-specific promoter; and
a gene of interest.

The Schwann cell-specific promoter is the same as the promoter described above in section "1. Schwann cell-specific promoter" and has the same properties and structure as configurations described in the section.

At this time, in one embodiment, the gene of interest may be a nucleic acid encoding a protein used for artificially engineering genes in Schwann cells. For example, the protein used for artificially engineering genes may be Cas protein, ZFN, or TALEN but is not limited thereto. Herein, the Cas protein may be Cas9 protein, Cas12a1 (Cpf1) protein, or Cas12f1 protein but are not limited thereto. By using this, a knock-out effect on genes associated with diseases related to Schwann cells may be achieved.

In addition, the gene of interest serves as a gene associated with diseases related to Schwann cells, so, if necessary, the promoter of the present disclosure may be used to overexpress the gene of interest in Schwann cells.

The gene associated with diseases related to Schwann cells may be a connexin 32 (Cx32) gene, SH3 domain and tetratricopeptide repeats 2 (SH3TC2) gene, myelin protein zero (MPZ) gene, early growth response 2 (EGR2) gene, ganglioside induced differentiation associated protein 1 (GDAP1) gene, N-Myc downstream regulated 1 (NDRG1) gene, or peripheral myelin protein 22 (PMP22) gene. To obtain disease treatment effects, the genes may be knocked out or overexpressed, if needed.

The Schwann cell-specific expression vector may further include a regulatory/control element. At this time, the regulatory/control component may be operably linked to the gene of interest included in the vector. The regulatory/control component may be an enhancer, artificial intron, termination signal, polyadenylation signal, Kozak consensus sequence, internal ribosome entry site (IRES), splice acceptor, 2A sequence, and/or replication origin, but is not limited thereto.

When the Schwann cell-specific expression vector includes a nucleic acid encoding a protein for artificially engineering genes in the Schwann cells, the composition may further include a guide RNA or nucleic acid encoding the guide RNA. At this time, the guide RNA may target genes that cause diseases related to Schwann cells. In another embodiment, the guide RNA may include a scaffold portion from which the poly-T portion has been removed. In yet another embodiment, the guide domain of the guide RNA may be a sequence that binds to a partial sequence of the pmp22 gene.

### 4-1-1. Composition example (1)

In one embodiment, the composition may include a Schwann cell-specific expression vector including the following:
a Schwann cell-specific promoter; and
a nucleic acid encoding Cas9 protein.

The Schwann cell-specific promoter is the same as the promoter described above in section "1. Schwann cell-specific promoter" and has the same properties and structure as configurations described in the section.

The composition may further include a guide RNA or a nucleic acid encoding the guide RNA.

In another embodiment, the composition may include one Schwann cell-specific expression vector selected in several embodiments described above in sections "2-1. Example of Schwann cell-specific expression vector (1)" and "2-2. Example of Schwann cell-specific expression vector (2)". At this time, the composition may further include a guide RNA or a nucleic acid encoding the guide RNA. In yet another embodiment, the guide RNA may include a scaffold portion from which the poly-T portion has been removed. In yet another embodiment, the guide domain of the guide RNA may be a sequence that binds to a partial sequence of the pmp22 gene.

### 4-1-2. Composition example (2)

In one embodiment, the composition may include a Schwann cell-specific expression vector including the following:
a Schwann cell-specific promoter;
a nucleic acid encoding Cas9 protein; and
a regulatory/control element.

The Schwann cell-specific promoter is the same as the promoter described above in section "1. Schwann cell-specific promoter" and has the same properties and structure as configurations described in the section.

The composition may further include a guide RNA or a nucleic acid encoding the guide RNA.

In another embodiment, the composition may include one Schwann cell-specific expression vector selected in several embodiments described above in sections "2-3. Example of Schwann cell-specific expression vector (3)" and "2-4. Example of Schwann cell-specific expression vector (4)". At this time, the composition may further include a guide RNA or a nucleic acid encoding the guide RNA. In yet another embodiment, the guide RNA may include a scaffold portion from which the poly-T portion has been removed. In yet another embodiment, the guide domain of the guide RNA may be a sequence that binds to a partial sequence of the pmp22 gene.

### 4-2. Treatment method

One aspect disclosed by the present disclosure relates to a method of treating diseases related to Schwann cells. The method uses a composition including a Schwann cell-specific expression vector. The Schwann cell-specific expression vector is the same as the expression vector described above in section "2. Schwann cell-specific expression vector" and has the same properties and structure as configurations described in the section. The Schwann cell-specific expression vector may include a gene associated with diseases related to Schwann cells and/or nucleic acid encoding a protein for artificially engineering genes in Schwann cells as a gene of interest included in the vector. Therefore, diseases related to Schwann cells may be treated by using the composition.

The method may comprise introducing (or administering) a composition to a subject.

At this time, the subject may be a human or non-human animal with diseases related to Schwann cells. The composition is the same as the composition described above in the section "4-1. composition" and has the same properties and structure as the configurations described in the section. For example, the diseases related to Schwann cells may include Charcot-Marie-Tooth (CMT) disease or motor neuron disease (MND) but are not limited thereto.

At this time, the introduction (or administration) of the composition to the subject may be performed by injection, transfusion, implantation, or transplantation. At this time, the introduction (or administration) of the composition to a subject may be performed through any one administration route selected from subretinal, subcutaneous, intradermal, intraocular, intravitreal, intratumoral, intranodal, intramedullary, intramuscularly, intravenous, intralymphatic, or intraperitoneal administration routes.

### 4-2-1. Treatment method example(1)

In one embodiment, the treatment method may comprise introducing or administering a composition to a subject.

At this time, the composition may include a Schwann cell-specific expression vector including the following:
a Schwann cell-specific promoter; and
a nucleic acid encoding Cas9 protein.

The Schwann cell-specific promoter is the same as the promoter described above in section "1. Schwann cell-specific promoter" and has the same properties and structure as configurations described in the section.

The composition may further include a guide RNA or a nucleic acid encoding the guide RNA. In another embodiment, the guide RNA may include a scaffold portion from which the poly-T portion has been removed. In yet another embodiment, the guide domain of the guide RNA may be a sequence that binds to a partial sequence of the pmp22 gene.

The subject may be a human or non-human animal with diseases associated with Schwann cells.

In yet another embodiment, the treatment method may comprise introducing (administering) a composition to a human subject having diseases related to Schwann cells. At this time, the composition may include one Schwann cell-specific expression vector selected in the embodiments described above in sections "2-1. Example of Schwann cell-specific expression vector (1)" and "2-2. Example of Schwann cell-specific expression vector (2)". At this time, the composition may further include a guide RNA or a nucleic acid encoding the guide RNA. At this time, the diseases related to Schwann cells may be Charcot-Marie-Tooth (CMT) disease.

### 4-2-2. Treatment method example (2)

In one embodiment, the treatment method may comprise introducing or administering a composition to a subject.

At this time, the composition may include a Schwann cell-specific expression vector including the following:
a Schwann cell-specific promoter;
a nucleic acid encoding Cas9 protein; and
a regulatory/control element.

The Schwann cell-specific promoter is the same as the promoter described above in section "1. Schwann cell-specific promoter" and has the same properties and structure as configurations described in the section.

The composition may further include a guide RNA or a nucleic acid encoding the guide RNA. In another embodiment, the guide RNA may include a scaffold portion from which the poly-T portion has been removed. In yet another embodiment, the guide domain of the guide RNA may be a sequence that binds to a partial sequence of the pmp22 gene.

The subject may be a human or non-human animal with diseases related to Schwann cells.

In yet another embodiment, the treatment method may comprise introducing (administering) a composition to a human subject having diseases related to Schwann cells. At this time, the composition may include one Schwann cell-specific expression vector selected in the embodiments described above in sections "2-3. Example of Schwann cell-specific expression vector (3)" and "2-4. Example of Schwann cell-specific expression vector (4)". At this time, the composition may further include a guide RNA or a nucleic acid encoding the guide RNA. At this time, the diseases related to Schwann cells may be Charcot-Marie-Tooth (CMT) disease.

However, the examples of composition and treatment method disclosed above are merely examples and are not limited thereto.

Hereinafter, the possible embodiments of the present disclosure provided in this specification are listed. The following embodiments provided in this paragraph are merely examples of the disclosure. Therefore, the invention provided in this specification cannot be interpreted as limited to the following examples. The brief description provided along with the example number is only for the convenience of distinguishing between each example and cannot be construed as a limitation on the disclosure disclosed in this specification.

### Possible examples of disclosure

### Schwann cell-specific promoter

### Example 1, Schwann cell-specific promoter

A Schwann cell-specific promoter which is an artificial promoter or engineered promoter with a sequence length in a range of 300 to 500 bp.

### Example 2, Limitation to length of promoter

The Schwann cell-specific promoter of example 1, wherein the Schwann cell-specific promoter has a sequence length in a range of 300 to 350 bp.

### Example 3, MPZ promoter origin

The Schwann cell-specific promoter of any one of examples 1 to 2, wherein the Schwann cell-specific promoter is an engineered version of the known human or rat MPZ promoter.

### Example 4, Limitation to engineering of MPZ promoter

The Schwann cell-specific promoter of any one of examples 1 to 3, wherein the Schwann cell-specific promoter is an artificially engineered Schwann cell-specific promoter with some nucleotide sequences of the known human or rat MPZ promoter deleted or with an IE sequence added to either end (5' and 3') of the known human or rat MPZ promoter.

### Example 5, Limitation to TATA box, CAAT box, or TSS

The Schwann cell-specific promoter of any one of examples 1 to 4, wherein the Schwann cell-specific promoter includes a TATA box, CAAT box, or transcription start site (TSS).

### Example 6, Limitation to promoter sequence (short form)

The Schwann cell-specific promoter of any one of examples 1 to 5, wherein the Schwann cell-specific promoter has a sequence selected from:

SEQ ID NO: 3; SEQ ID NO: 4; SEQ ID NO: 5; SEQ ID NO: 6; and a sequence having at least 90% homology with one of SEQ ID NOs: 3, 4, 5, and 6.

### Example 7, Limitation to sequence length of 300bp

The Schwann cell-specific promoter of example 6, wherein the Schwann cell-specific promoter has a sequence shown in SEQ ID NO: 3 or a sequence having at least 90% homology with SEQ ID NO: 3.

### Example 8, IE addition to promoter

The Schwann cell-specific promoter of any one of examples 1 to 7, wherein the Schwann cell-specific promoter further includes an intronic enhancer (IE) at the 3' or 5' end.

### Example 9, Limitation to IE sequence

The Schwann cell-specific promoter of example 8, wherein the sequence of IE is a sequence shown in SEQ ID NO: 7 or a sequence having at least 90% homology with SEQ ID NO: 7.

### Example 10, Limitation to sequence (IE added form)

The Schwann cell-specific promoter of any one of examples 8 to 9, wherein the Schwann cell-specific promoter consists of a sequence selected from:

SEQ ID NO: 8; SEQ ID NO: 9; SEQ ID NO: 11; SEQ ID NO: 12; SEQ ID NO: 13; SEQ ID NO: 14; SEQ ID NO: 15; SEQ ID NO: 16; and a sequence having at least 90% homology with one of SEQ ID NOs: 8, 9, 11, 12, 13, 14, 15 and 16.

### Example 11, Limitation to sequence length of 417bp 1

The Schwann cell-specific promoter of any one of example 10, wherein the Schwann cell-specific promoter consists of a sequence shown in SEQ ID NO: 8 or a sequence having at least 90% homology with SEQ ID NO: 8.

### Example 12, Limitation to sequence length of 417bp 2

The Schwann cell-specific promoter of any one of example 10, wherein the Schwann cell-specific promoter consists of a sequence shown in SEQ ID NO: 9 or a sequence having at least 90% homology with SEQ ID NO: 9.

### Example 13, Limitation to sequence length of 317bp

The Schwann cell-specific promoter of any one of example 10, wherein the Schwann cell-specific promoter consists of a sequence shown in SEQ ID NO: 13 or a sequence having at least 90% homology with SEQ ID NO: 13.

### Example 14, Emphasis on three-fold difference in length

The Schwann cell-specific promoter of any one of examples 7 to 13, wherein the Schwann cell-specific promoter has 1/3 or less the length of the known MPZ promoter.

### Schwann cell-specific expression vector

### Example 15, Inclusion of Schwann cell-specific promoter

The Schwann cell-specific expression vector comprising the Schwann cell-specific promoter of any one of examples 1 to 14.

### Example 16, Inclusion of gene of interest

The Schwann cell-specific expression vector of example 15, wherein the Schwann cell-specific expression vector further includes a gene of interest, and the gene of interest is operably linked to the Schwann cell-specific promoter.

### Example 17, Limitation to gene of interest (treatment of diseases related to Schwann cells)

The Schwann cell-specific expression vector of example 16, wherein the gene of interest is a gene associated with the treatment of diseases related to Schwann cells.

### Example 18, Limitation to diseases related to Schwann cells

The Schwann cell-specific expression vector of example 17, wherein the gene associated with diseases related to Schwann cells is a connexin 32 (Cx32) gene, SH3 domain and tetratricopeptide repeats 2 (SH3TC2) gene, myelin protein zero (MPZ) gene, early growth response 2 (EGR2) gene, ganglioside induced differentiation associated protein 1 (GDAP1) gene, N-Myc downstream regulated 1 (NDRG1) gene, or peripheral myelin protein 22 (PMP22) gene.

### Example 19, Limitation to gene of interest (protein for artificially engineering genes)

The Schwann cell-specific expression vector of example 16, wherein the gene of interest is a gene encoding a protein for artificially engineering genes in Schwann cells.

### Example 20, Limitation to protein for artificially engineering genes

The Schwann cell-specific expression vector of example 19, wherein the protein for artificially engineering genes is a Cas9 protein, Cas12a1 (Cpf1) protein, Cas12f1 protein, Zinc Finger Nuclease (ZFN), or Tranion Activator-Like Effector Nuclease (TALEN) .

### Example 21, Inclusion of regulatory/control element

The Schwann cell-specific expression of any one of examples 15 to 20, the Schwann cell-specific expression vector further includes a regulatory/control element.

### Example 22, Example of regulatory/control element

The Schwann cell-specific expression vector of example 21, wherein the regulatory/control element includes one or more selected from a polyadenylation signal, Woodchuck hepatitis virus posttranscriptional regulatory element (WPRE), and artificial intron.

### Example 23, Limitation to polyadenylation signal

The Schwann cell-specific expression vector of example 22, wherein the polyadenylation signal is a bovine growth hormone polyadenylation signal (BGHA), synthetic polyadenylation signal (synpA), or simian virus 40 polyadenylation signal (SV40pA).

### Example 24, Limitation to SV40pA and synpA sequences

The Schwann cell-specific expression vector of example 23, wherein the SV40pA has any one selected from SEQ ID NO: 17 and a nucleotide sequence having at least 90% homology with SEQ ID NO: 17, or the synpA has any one selected from SEQ ID NO: 18 and a nucleotide sequence having at least 90% homology with SEQ ID NO: 18.

### Example 25, Limitation to WRPE

The Schwann cell-specific expression vector of example 22, wherein the WPRE includes one or more selected from gamma, alpha, and beta elements.

### Example 26, Limitation to WRPE sequence

The Schwann cell-specific expression vector of example 25, wherein the WPRE has a nucleotide sequence selected from SEQ ID NO: 19; SEQ ID NO: 20, and a nucleotide sequence having at least 90% homology with SEQ ID NO: 19 or SEQ ID NO: 20.

### Example 27, Limitation to artificial intron

The Schwann cell-specific expression vector of example 22, wherein the artificial intron is for improving the expression of the gene of interest in the Schwann cell-specific expression vector.

### Example 28, Limitation to artificial intron sequence

The Schwann cell-specific expression vector of example 27, wherein the artificial intron has a nucleotide sequence selected from a nucleotide sequence of SEQ ID NO: 21 and a nucleotide sequence having at least 90% homology with SEQ ID NO: 21.

### Example 29, Limitation to vector

The Schwann cell-specific expression vector of any one of examples 15 to 28, the Schwann cell-specific expression vector is a viral vector.

### Example 30, Limitation to vector type

The Schwann cell-specific expression vector of example 29, wherein the viral vector is a viral vector selected from the group consisting of a retroviral (retrovirus) vector, lentiviral (lentivirus) vector, adenoviral (adenovirus) vector, adeno-associated viral (adeno-associated virus; AAV) vector, vaccinia viral (vaccinia virus) vector, poxviral (poxvirus) vector, and herpes simplex viral (herpes simplex virus) vector.

### Composition including Schwann cell-specific expression vector

### Example 31, Composition including Schwann cell-specific expression vector

A composition comprising the Schwann cell-specific expression vector of any one of examples 15 to 30.

### Example 32, Composition for artificially engineering genes

The composition of example 31, wherein the gene of interest is a nucleic acid encoding Cas9 protein, Cas12a1 (Cpf1) protein, Cas12f1 protein, Zinc Finger Nuclease (ZFN), or Tranion Activator-Like Effector Nuclease (TALEN).

### Example 33, Addition of guide RNA

The composition of example 32, wherein the composition further includes a guide RNA or a nucleic acid encoding the guide RNA.

### Example 34, Properties of guide RNA

The composition of example 33, wherein the guide RNA targets genes causing diseases related to Schwann cells.

### Example 35, Limitation to target gene

The composition of example 34, wherein the gene causing diseases related to Schwann cells is a connexin 32 (Cx32) gene, SH3 domain, and tetratricopeptide repeats 2 (SH3TC2) gene, myelin protein zero (MPZ) gene, early growth response 2 (EGR2) gene, ganglioside induced differentiation associated protein 1 (GDAP1) gene, N-Myc downstream regulated 1 (NDRG1) gene, or peripheral myelin protein 22 (PMP22) gene.

### Example 36, Addition of carrier

The composition of any one of examples 31 to 34, wherein the composition comprises pharmaceutically acceptable carrier comprising one or more selected from the group consisting of binders (for example, lactose, saccharose, sorbitol, mannitol, starch, amylopectin, cellulose, or gelatin); excipients (for example, dicalcium phosphate); disintegrants (for example, corn starch or sweet potato starch); lubricants (for example, magnesium stearate, calcium stearate, sodium stearyl fumarate, or polyethylene glycol wax); sweetener; air freshener; syrup; liquid carriers (for example, fatty oils); sterile aqueous solution; propylene glycol; polyethylene glycol; injectable esters (for example, ethyl oleate); Suspension agent; emulsion; freeze-dried preparation; topical preparation; stabilizer; buffering agent; animal oil; vegetable oil; wax; paraffin; starch; tragacant; cellulose derivatives; polyethylene glycol; silicon; bentonite; silica; talc; zinc oxide; and mixtures thereof.

### Example 37, Composition for treatment

The composition of any one of Examples 31 to 36 used to treat, or is intended to treat, diseases related to Schwann cells.

### Method for specifically expressing gene of interest or protein of interest in Schwann cells

### Example 38, Method using Schwann cell-specific expression vector

A method of specifically expressing a gene of interest or protein of interest in Schwann cell using the Schwann cell-specific expression vector of any one of Examples 15 to 30.

### Example 39, addition of method of delivering vectors to cells

The method of specifically expressing a gene of interest or protein of interest in the Schwann cell of example 38, wherein the method comprises introducing or delivering the Schwann cell-specific expression vector into the Schwann cell.

### Example 40, Limitation to delivery method

The method of specifically expressing a gene of interest or protein of interest in the Schwann cell of example 39, wherein the method of introducing or delivering the Schwann cell-specific expression vector to the Schwann cell is performed by a cationic liposome method, lithium acetate-DMSO, lipid-mediated transfection, calcium phosphate precipitation, lipofection, polyethyleneimine (PEI)-mediated transfection, DEAE-dextran-mediated transfection, nanoparticle-mediated nucleic acid delivery, electroporation method, gene gun, sonoporation, magnetofection method, nanoparticle method, and/or temporary cell compression or squeezing method.

### Example 41, Limitation to Schwann cells

The method of specifically expressing a gene of interest or protein of interest in Schwann cell of any one of examples 38 to 40, wherein the Schwann cell is human Schwann cell or non-human animal Schwann cell.

### Example 42, Limitation to experiment environment such as in vivo

The method of specifically expressing a gene of interest or protein of interest in Schwann cells of any one of examples 38 to 41, the introducing or delivering of the Schwann cell-specific expression vector to the Schwann cell is performed in vitro, ex vivo, or in vivo.

**Uses in treating diseases related to Schwann cells**

### Example 43, Treatment method

A method for treating disease related to Schwann cells comprising introducing (or administering) the composition of any one of examples 31 to 37 into a subject.

### Example 44, Limitation to administration method

The method for treating disease related to Schwann cells of example 44, wherein the introduction (or administration) of the composition to the subject is performed by injection, transfusion, implantation, or transplantation methods.

### Example 45, Limitation to administration routes

The method for treating disease related to Schwann cells of any one of examples 43 to 44, the administration of the composition to the subject is performed by any administration route selected from subretinal, subcutaneous, intradermal, intraocular, intravitreal, intratumoral, intranodal, intramedullary, intramuscular, intravenous, intralymphatic, or intraperitoneal administration routes.

### Example 46, Limitation to diseases

The method for treating disease related to Schwann cells of any one of examples 43 to 45, the disease related to Schwann cells is Charcot-Marie-Tooth (CMT) disease or motor neuron disease (MND).

### Mode for Disclosure

The present disclosure will be described in detail below through experimental examples.

These experimental examples are only for illustrating the present disclosure in more detail, and the experimental examples will be apparent to those skilled in the art that the scope of the present disclosure is not limited by these experimental examples.

### Experimental examples

### Experimental methods

### 1. Plasmid generation

pAAV-SpCas9-related vectors were constructed by cloning using Gibson assembly. The vectors were constructed by positioning codon-optimized SpCas9 or codon-optimized sauriCas9 sequences, and each of the to-be-compared promoter sequences of interest (SEQ ID NOs: 3, 7, 8, 13, and 32) between the inverted tandem repeats (ITR) of AAV2 or AAV9. And each of the to-be-compared promoter sequences of interest was on the Cas9 upstream. The to-be-compared promoter sequences included variations: a promoter in which a 300 bp small MPZ promoter (sMPZ promoter, SEQ ID NO: 3); a promoter (SEQ ID NO: 8) in which a 117 bp egr2/sox10-binding site (Intronic Enhancer or IE, SEQ ID NO: 7) were added upstream of the sMPZ promoter; a promoter (SEQ ID NO: 13) in which the 117 bp egr2/sox10-binding site was added downstream of the sMPZ promoter; a promoter (SEQ ID NO: 9) in which the portion of the 5' end of the sMPZ promoter was replaced with IE; or an EFS promoter (non-specific to Schwann cells) previously known to have a high expression rate in all tissues.

Based on the constructed vectors, a vector including synpA and sv40pA instead of BGHA was further constructed using Gibson assembly. The EPS promoter had the following nucleic acid sequence:

GGGCAGAGCGCACATCGCCCACAGTCCCCGAGAAGTTGGGGGGAGGGGTCGGCAATTGATCCG
GTGCCTAGAGAAGGTGGCGCGGGGTAAACTGGGAAAGTGATGTCGTGTACTGGCTCCGCCTTT
TTCCCGAGGGTGGGGGAGAACCGTATATAAGTGCAGTAGTCGCCGTGAACGTTCTTTTTCGCA
ACGGGTTTGCCGCCAGAACACAG-3′(SEQ ID NO: 32).

pAAV-U6-sgRNA vectors carrying U6 promoter and sgRNA (unmodified or sf-modified) sequences between inverted tandem repeats (ITR) were constructed. SpCas9-specific guide RNAs for pmp22-TATA (guide RNA1 or guide RNA2 in Table 1) were produced by oligo cloning using the BspQI site.

For testing SauriCas9, single vectors were constructed in the form of pAAV-SauriCas9-U6-sgRNA vectors, which included both SauriCas9 and sgRNA sequences positioned between ITRs. At this time, in the vectors, each of the to-be-compared promoters was positioned upstream of SauriCas9, a mvm intron was placed between each of the promoters and SauriCas9 sequences, and a WPRE-short was positioned between SauriCas9 and sv40pA. In addition, a sgRNA backbone was used as the backbone of *Staphylococcus aureus* Cas9 (SaCas9), which has proven its activity. SauriCas9-specific guide RNA for pmp22-TATA (Table 1) was produced by oligo cloning using the BspQI site.

In addition, pAAV-tdTomato-related vectors were constructed by cloning using Gibson assembly. The vectors were constructed by positioning codon-optimized tdTomato sequences and each of the to-be-compared promoter sequences of interest (SEQ ID NOs: 1, 3, and 9) between the inverted tandem repeats (ITR) of AAV9. And each of the to-be-compared promoter sequences of interest was positioned on the upstream of the tdTomato. The to-be-compared promoter sequences of interest included variations: a 300 bp small MPZ promoter (sMPZ promoter, SEQ ID NO: 3); a promoter (SEQ ID NO: 9) in which the portion of the 5' end of the sMPZ promoter was replaced with IE; and a known MPZ promoter (SEQ ID NO: 1) . The tdTomato had the following nucleic acid sequence:

TGAACTTCGAGGACGGCGGTCTGGTGACCGTGACCCAGGACTCCTCCCTGCAGGACGGCACGC
TGATCTACAAGGTGAAGATGCGCGGCACCAACTTCCCCCCCGACGGCCCCGTAATGCAGAAGA
AGACCATGGGCTGGGAGGCCTCCACCGAGCGCCTGTACCCCCGCGACGGCGTGCTGAAGGGCG
AGATCCACCAGGCCCTGAAGCTGAAGGACGGCGGCCACTACCTGGTGGAGTTCAAGACCATCT
ACATGGCCAAGAAGCCCGTGCAACTGCCCGGCTACTACTACGTGGACACCAAGCTGGACATCA
CCTCCCACAACGAGGACTACACCATCGTGGAACAGTACGAGCGCTCCGAGGGCCGCCACCACC
TGTTCCTGTACGGCATGGACGAGCTGTACAAGTAG-3′(SEQ ID NO: 33).

**[Table 1] Guide RNA sequence used**

| Target Sites | Target Sequence with PAM (5'-3') | SEQ ID NC |
|---|---|---|
| Sp-PMP22-TATA(guide RNA1) | GGACCAGCCCCTGAATAAAC-TGG | 22 |
| Sauri-PMP22-TATA | ATTCAGGGGCTGGTCCAATGC-TGGG | 23 |
| Sp-PMP22-TATA(guide RNA2) | GACCAGCCCCTGAATAAAC-TGG | 34 |

### 2. Cell culture and CRISPR/Cas9(or tdTomato) transfection

Human Schwann-like cell line (sNF02.2) (CRL-2885, ATCC), S16 cell line (CRL-2941, ATCC), and RT4 cell line (CRL2768, ATCC) were maintained by subculturing every week with Dulbecco's Modified Eagle Medium (DMEM) (WelGene) supplemented with 10% fetal calf serum (WelGene) and 1X penicillin/streptomycin (WelGene) .

pAAV-SpCas9-related vectors and pAAV-U6-sgRNA-related vectors, each at a concentration of 0.1 pmol, were co-transfected into Human Schwann-like cells (sNF02.2). Neon transfection (Thermo Fisher) was used, and the transfection was performed by placing 1.5×10⁵ cells in the following conditions: 10 µl tip, 1400 V, 20 ms, and 2 pulses. Forkskolin was applied at a concentration of 2.5 µM to treat the cells, cell pellets were obtained 5 days after the transfection, and RNA was extracted using a Quick DNA/RNA Miniprep Kit (ZYMO RESEARCH).

pAAV-SpCas9-related vectors and pAAV-U6-sgRNA-related vectors, each at a concentration of 0.1 pmol, were co-transfected into S16 cells. The transfection was performed by placing 1.5×10⁵ cells in the following conditions: 10 µl tip, 1400 V, 20 ms, and 2 pulses. Cell pellets were obtained 3 days after the transfection, and RNA was extracted using the Quick DNA/RNA Miniprep Kit (ZYMO RESEARCH).

pAAV-SpCas9-related vectors and pAAV-U6-sgRNA-related vectors, each at a concentration of 0.1 pmol, were co-transfected into RT4-D6P2T cells. The transfection was performed by placing 1.5×10⁵ cells in the following conditions: 10 µl tip, 1400 V, 20 ms, and 2 pulses. Cell pellets were obtained 3 days after the transfection, and RNA was extracted using the Quick DNA/RNA Miniprep Kit (ZYMO RESEARCH).

RT4-D6P2T cells were seeded into a 12-well plate at a density of 5×10⁵ cells per well. AAV stock was thawed and diluted to 1×10⁴ per cell. A total of 100 µl of AAV was added to each well of the plate at a concentration of 1×10⁴ per cell and then cultured for 3 days. Next, genomic DNA was extracted and then the on-target site was amplified by PCR. For next-generation sequencing, PCR was further performed by attaching a specific adapter and TruSeq HT Dual Index primers to the sequencing primer. Afterward, by using paired sequencing reads, the activity of guide RNA1 (SEQ ID NO: 22) and guide RNA2 (SEQ ID NO: 34) was evaluated through quantitative analysis of insertions or deletions (Indels) at an on-target genomic location.

RT4-D6P2T cell suspension was made at a density of 6.25×10⁵ cells/ml, and 800 ul of cell suspension was inoculated into each well. The cells were distributed and cultured at 5×10⁵ cells/well (12-well plate). pAAV-tdTomato-related vectors were constructed at a concentration of 1×10⁶ per cell. The cells were treated with 100 µl of the pAAV-tdTomato-related vector at a density of 1×10⁶ gc/cell and then cultured. DNA and RNA were extracted from the cultured cells using the Quick DNA/RNA Miniprep Kit (ZYMO RESEARCH) . Alternatively, the cultured cells were placed in a 1.5 ml tube and then centrifuged at 9000 rpm for 2 minutes at a temperature of 4°C. The supernatant from the centrifugation was removed, and 1 mL of FACS buffer was added to wash the cells. The cells were centrifuged at 9000 rpm for 2 min at a temperature of 4°C. The supernatant was aspirated and resuspended in 300 ul of FACS buffer. Next, an analysis was performed on a FACS device. pAAV-SpCas9-related vectors and pAAV-U6-sgRNA-related vectors, each at a concentration of 0.1 pmol, were co-transfected into RT4 cells. Alternatively, pAAV-SauriCas9-U6-sgRNA-related vectors were transfected at a concentration of 0.2 pmol into the RT4 cells. The transfection was performed by placing 2×10⁵ cells in the following conditions: 10 µl tip, 1400 V, 20 ms, and 2 pulses. Cell pellets were obtained 3 days after the transfection, and DNA and RNA were extracted using a Quick DNA/RNA Miniprep Kit (ZYMO RESEARCH).

The pAAV-SpCas9-related vectors and the pAAV-U6-sgRNA-related vectors were mixed 1:1 to a total of 1×10¹⁴ vg/kg and administered to the tail vein of anesthetized experimental animals (rats). After 4 weeks, the experimental animals (rats) were sacrificed, and then the liver, sciatic nerve, and lumbar nerve thereof were collected. From there, DNA and RNA were extracted using the Quick DNA/RNA Miniprep Kit (ZYMO RESEARCH).

### 3. Real time PCR (qRT-PCR)

The extracted RNA was adjusted to a total amount of 100 to 1000 ng, and then the RNA was used to manufacture cDNA using a cDNA Reverse Transcription kit (Thermo Fisher). qRT-PCR was performed on a QuantStudio 3 instrument using 10 to 15 ng of cDNA with SYBR Green Master Mix in accordance with the manufacturer's protocol (Thermo Fisher). The expression levels of SpCas9 were calculated using CT values, with hGAPDH and rGAPDH used as internal controls. The primers used are shown in Table 2.

**[Table 2] q-RT primer used**

| Primer name | Sequence (5'-3') | SEQ ID NC |
|---|---|---|
| SpCas9_F | GGACTTCTACCCCTTCCTGA | 24 |
| SpCas9_R | GTGATGGTCTCCTCGCTCT | 25 |
| hGAPDH_F | GTCTCCTCTGACTTCAACAGCC | 26 |
| hGAPDH_R | ACCACCCTGTTGCTGTAGCCAA | 27 |
| rGAPDH_F | GAAACCCATCACCATCTTCC | 28 |
| rGAPDH_R | GTTCACACCCATCACAAACAT | 29 |

### 4. Indel analysis (Targeted deep- sequencing)

The on-target site was amplified using the extracted genomic DNA and primers (Table 3), and then a barcode was created per sample through additional PCR using the illumine TrueSeq adapter (Illumina) . Each sample was purified using a PCR purification kit (Intronbio). After pooling the samples in an equimolar ratio, paired sequencing was performed using Miseq and TrueSeq (HT) dual index system (Illumina) to produce sequencing reads. The generated reads were analyzed using the Cas9-Analyzer tool from CRISPR/RGEN Tools (www.rgenome.net) to quantitatively calculate insertion or deletion (Indels) reads at on-target genomic locations. This allowed for comparing and evaluating the gene editing efficiency mediated by the vectors.

**[Table 3] targeted deep sequencing primer used**

| Primer name | Sequence (5'-3') | SEQ ID NC |
|---|---|---|
| rPMP22_F | TCCAAGTTCATTTCCTGCAGG | 30 |
| rPMP22_R | CCAGGCTCCCTGAGATGTTC | 31 |

### 5. Statistics

For statistical analysis, a student t-test was used. Significance means * means p<0.05, ** means p<0.01, and *** means p<0.001.

### Experimental results

### Experimental Example 1. Effect of engineered MPZ promoters in rat Schwann cell line and rat Schwann-like cell line

### Experimental Example 1-1. Effects in Rat Schwann cell line - S16 cells

To engineer the small MPZ promoter (sMPZ, SEQ ID NO: 3) including the transcription start site (TSS) and TATA box of the rat MPZ gene, a vector which expresses SpCas9 according to a promoter (IE-sMPZ, SEQ ID NO: 8) in which a 117bp intronic enhancer (IE, SEQ ID NO: 7) were added upstream of the sMPZ promoter; a vector which expresses SpCas9 according to a promoter (sMPZ-IE, SEQ ID NO: 13) in which the IE were added downstream of the sMPZ promoter; and a vector which expresses SpCas9 according to a promoter (IE200, SEQ ID NO: 9) in which the portion of the 5' end of the sMPZ promoter was replaced with IE were constructed. The IE was positioned within the first intron of the MPZ gene as a major egr2/sox10-binding site. Afterward, vectors expressing sgRNA targeting the pmp22-TATA region (guide RNA1 shown in Table 1) were co-transfected into S16 cells, which was a Schwann cell line, and then the expression and gene editing efficiency of SpCas9 were tested.

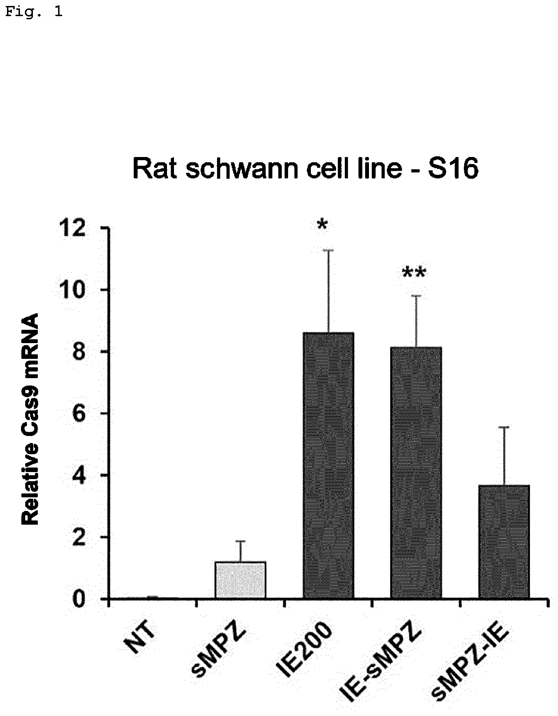

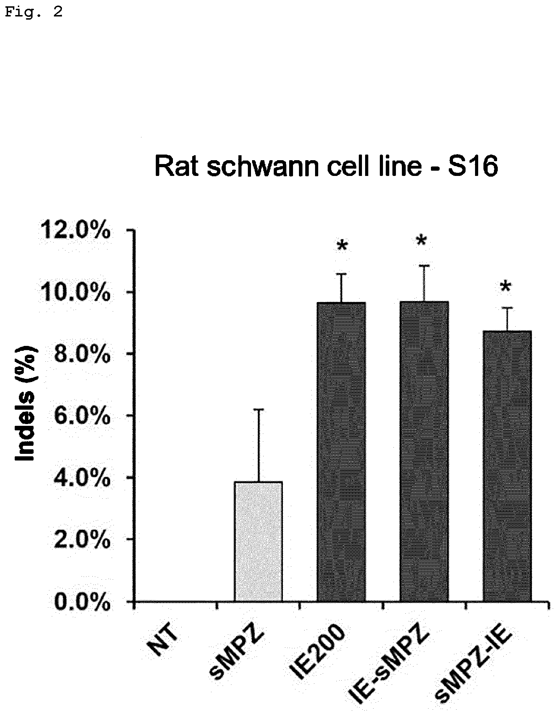

The results are shown in FIGs. 1 and 2.

FIG. 1 shows the results of confirming Cas9 expression after co-transfection of pAAV-SpCas9-BHGA expression vector and pAAV-U6-sgRNA expression vector in S16 cells (N=3-4). FIG. 2 shows the results of verifying the gene editing efficiency (B) for the pmp22-TATA target after co-transfecting the pAAV-SpCas9-BHGA expression vector and the pAAV-U6-sgRNA expression vector in S16 cells (N=3-4). At this time, NT mean non-transfection; sMPZ mean a small MPZ core promoter (SEQ ID NO: 3); IE mean an intronic enhancer (SEQ ID NO: 7); IE200 mean an engineered promoter (SEQ ID NO: 9); IE-sMPZ mean an engineered promoter (SEQ ID NO: 8); and sMPZ-IE mean an engineered promoter (SEQ ID NO: 13) . *, p<0.05 vs sMPZ; **, p<0.01 vs sMPZ.

As shown in FIG. 1, the expression of SpCas9 and indels occurring at the pmp22-TATA target site were confirmed in cells transfected with sMPZ promoter vector, IE200 vector, IE-sMPZ vector, and sMPZ-IE promoter vector. In other words, all promoters of the present disclosure might effectively express the gene of interest. In particular, the expression of SpCas9 was approximately 2 to 4 times higher in cells transfected with IE200, IE-sMPZ, and sMPZ-IE promoter vectors compared to cells transfected with the sMPZ promoter vector (FIG. 1).

Furthermore, regarding the gene editing effects mediated by the expressed SpCas9, indels occurring at the pmp22-TATA target site increased by 2-fold or more (FIG. 2).

### Experimental Example 1-2. Effects in Rat Schwann-like cell - RT4-D6P2T cells

The present inventors confirmed the efficacy of the promoter of the present disclosure using another Schwann-like cell line, which was RT4-D6P2T, in addition to the S16 cells used in Experimental Example 1-1.

RT4-D6P2T cells, which was a Schwann-like cell line, were co-transfected with vectors expressing SpCas9 under the control of the sMPZ promoter, IE200 promoter, IE-sMPZ promoter, and sMPZ-IE promoter and vectors expressing sgRNA (Guide RNA1 shown in Table 1) targeting the pmp22-TATA region. Subsequently, the gene editing efficiency was tested.

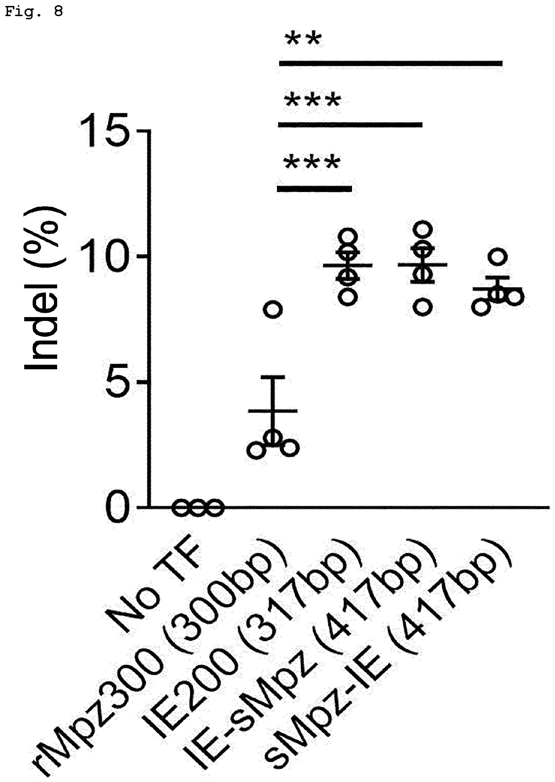

The results are shown in FIG. 8. FIG. 8 shows the results of verifying the gene editing efficiency for the pmp22-TATA target after co-transfection of the pAAV-SpCas9-BHGA expression vector and the pAAV-U6-sgRNA expression vector in RT4-D6P2T cells. At this time, NT mean non-transfection; rMPZ mean a small MPZ core promoter (SEQ ID NO: 3); IE mean an intronic enhancer (SEQ ID NO: 7); IE200 mean an engineered promoter (SEQ ID NO: 9); IE-sMPZ mean an engineered promoter (SEQ ID NO: 8); and sMPZ-IE mean an engineered promoter (SEQ ID NO: 13) . ** or *** means that the gene editing efficiency in sMpz300 was significantly different from the gene editing efficiency in IE200, IE-sMpz, and sMpz-IE.

As a result of experiments, indels occurring at the pmp22-TATA target site were observed in cells transfected with the sMPZ promoter vector, IE200 vector, IE-sMPZ vector, and sMPZ-IE promoter vector. In particular, 2-fold or more indels occurring at the pmp22-TATA target site were confirmed in cells transfected with the IE200, IE-sMPZ, and sMPZ-IE promoter vectors compared to cells transfected with the sMPZ promoter vector. The experimental results show that the promoters of the present disclosure might effectively express the target protein in Schwann cells and Schwann-like cells.

### Experimental Example 1-3. Use of guide RNAs with target sequences of different lengths

The present inventors utilized guide RNA and SpCas9 expressed under the control of IE200 promoter. Thus, the inventors confirmed the gene editing efficiency on target sequences of different lengths and whether the expressed CRISPR system functioned properly,

To the end, vectors expressing SpCas9 under the control of the IE200 promoter and vectors expressing sgRNA targeting the pmp22-TATA region (guide RNA1 or guide RNA2 shown in Table 1) were co-transfected into RT4-D6P2T cells, which was a Schwann-like cell line. Subsequently, the gene editing efficiency was tested. At this time, the guide RNA1 had a 20-nucleotide guide domain (a sequence binding to a partial sequence in the pmp22 gene) and the guide RNA2 had a 19-nucleotide guide domain (a sequence binding to a partial sequence in the pmp22 gene).

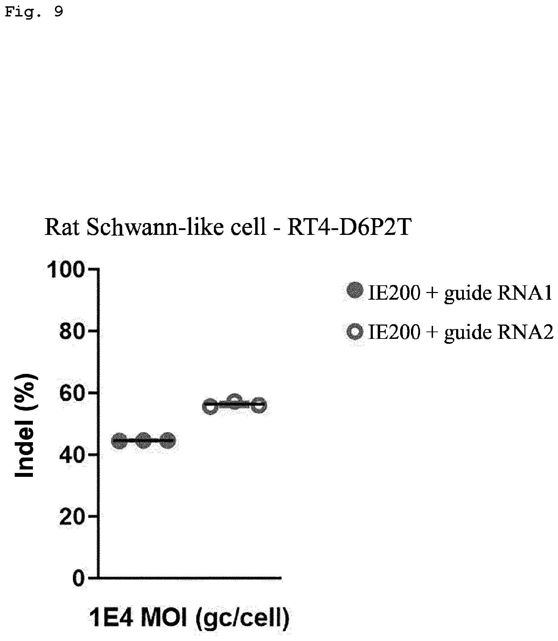

The results are shown in FIG. 9. FIG. 9 shows the results of verifying the gene editing efficiency for the pmp22-TATA target after co-transfection of the pAAV-SpCas9-BHGA expression vector and the pAAV-U6-sgRNA expression vector in RT4-D6P2T cells. At this time, IE200 was an engineered promoter (SEQ ID NO: 9). IE200+guide RNA1 meant the case after co-transfect the pAAV-SpCas9-BHGA expression vector for IE200 and the pAAV-U6-sgRNA expression vector for guide RNA1. IE200+guide RNA2 meant the case after co-transfect the pAAV-SpCas9-BHGA expression vector for IE200 and the pAAV-U6-sgRNA expression vector for guide RNA2.

As a result of the experiments, when using guide RNA1 and guide RNA2, similar levels of gene editing efficiency were confirmed, indicating that the expressed CRISPR system functioned effectively (FIG. 9).

The experimental results indicated that the SpCas9 expressed under the control of the engineered MPZ promoters of this present disclosure performed gene editing functions effectively.

### Experimental Example 1-4. Expression rate of tdTomato (fluorescent protein)

The present inventors also sought to confirm the expression levels of fluorescent proteins as the proteins of interest.

A vector expressing tdTomato under the control of the sMPZ promoter (300 bp), IE200 promoter (317 bp), and the known MPZ promoter (1000 bp) was transfected into RT4-D6P2T cells, which was a Schwann-like cell line, to test the expression levels of tdTomato (fluorescence intensity).

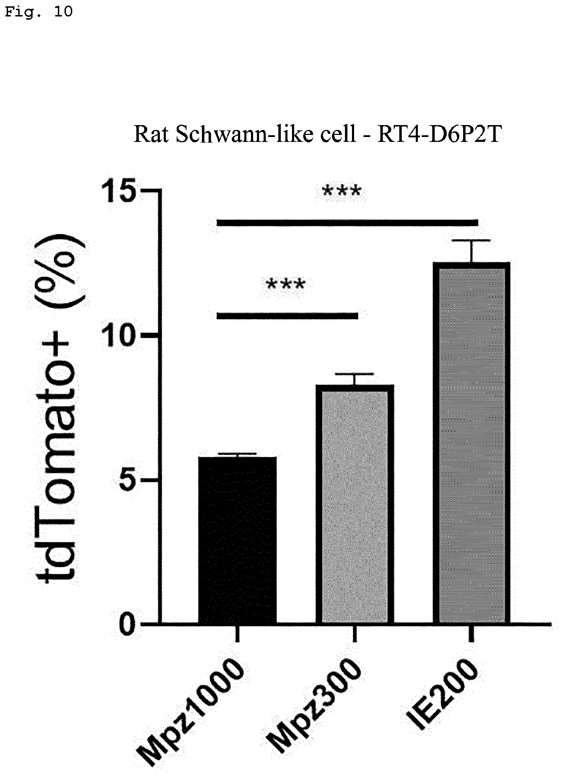

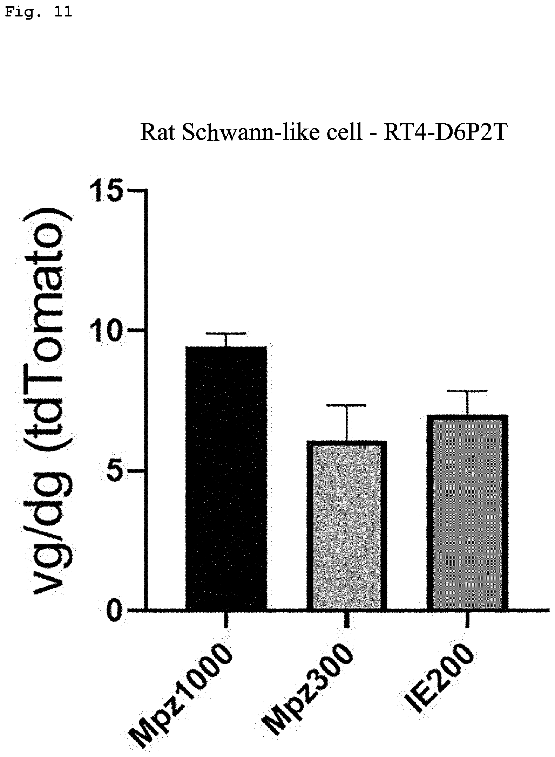

The results are shown in FIGs. 10 and 11. FIG. 10 shows the results of expression levels (fluorescence intensity) of the fluorescent protein (tdTomato) after transfection of the pAAV-tdTomato expression vector into RT4-D6P2T cells. At this time, MPZ1000 mean an MPZ promoter (SEQ ID NO: 1); MPZ300 mean a small MPZ core promoter (SEQ ID NO: 3); and IE200 mean an engineered promoter (SEQ ID NO: 9). *** means that the expression rate in Mpz1000 was significantly different from the gene editing efficiency in IE200 and Mpz300. FIG. 11 shows the results of confirming uniform cellular transformation after transfection of the pAAV-tdTomato expression vector into RT4-D6P2T cells. At this time, MPZ1000 mean an MPZ promoter (SEQ ID NO: 1); MPZ300 mean a small MPZ core promoter (SEQ ID NO: 3); and IE200 mean an engineered promoter (SEQ ID NO: 9).

As shown in FIG. 11, the known MPZ promoter was transformed to cells with similar efficiency as sMPZ promoter and IE200 promoter of the present disclosure. At this time, vg meant vector genome, and dg meant diploid genome.

As shown in FIG. 10, the sMPZ promoter and IE200 promoter of the present disclosure exhibited higher efficiency in expressing fluorescent proteins compared to the known MPZ promoter. In general, it was expected that using the 1000 bp-long known MPZ promoter would result in higher expression efficiency of the protein of interest. However, as a result of experiments, it was confirmed that the promoters of the present disclosure, which were approximately 1/3 the length of the known MPZ promoter, exhibited much higher efficiency in expressing fluorescent proteins. In particular, the IE200 promoter exhibited more than twice the expression level of the protein of interest compared to the known MPZ promoter.

The experimental results demonstrated that the Schwann cell-specific promoters of the present disclosure, despite being much shorter in length compared to the known MPZ promoter, might significantly enhance the expression of the protein of interest in Schwann cells at a much higher efficiency than the known MPZ promoter.

### Experimental Example 2. Effects of engineered MPZ promoters in human Schwann cell line

The present inventors sought to confirm the effects of the Schwann cell-specific promoters of the present disclosure in human Schwann cells.

Cas9 expression was verified after co-transfection of pAAV-SpCas9-BHGA expression vector and pAAV-U6-sgRNA expression vector in sNF02.2, which was a human Schwann cell line.

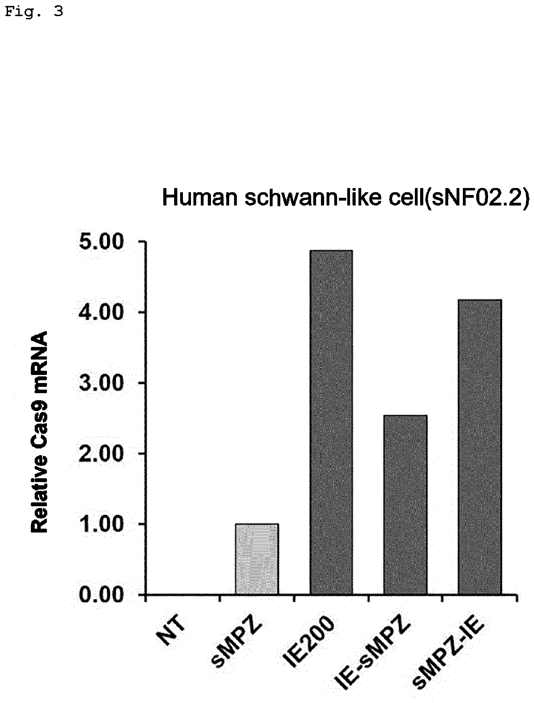

The results are shown in FIG. 3. FIG.3 shows the results of verifying Cas9 expression after co-transfection of pAAV-SpCas9-BHGA expression vector and pAAV-U6-sgRNA expression vector in sNF02.2, which was a human Schwann cell line. Cas9 expression was expressed as mean value (N=3) . At this time, NT meant non-transfection; sMPZ mean a small MPZ core promoter (SEQ ID NO: 3); IE200 mean an engineered promoter (SEQ ID NO: 9); IE-sMPZ was mean engineered promoter (SEQ ID NO: 8); and sMPZ-IE mean an engineered promoter (SEQ ID NO: 13).

As shown in FIG. 3, it was confirmed that SpCas9 was effectively expressed in NF02.2 cells by the Schwann cell-specific promoters of the present disclosure. In particular, among the three engineered promoters of the present disclosure, it was confirmed that the SpCas9 expression rate by the IE200 promoter was the highest (FIG. 3).

The experimental results show that the promoters of the present disclosure may effectively express the protein of interest even in human Schwann cells. In other words, this suggests that the Schwann cell-specific promoters of the present disclosure may be used for purposes such as human gene editing and disease treatment.

### Experimental Example 3. Additional modifications to expression system

The present inventors focused on experiments to confirm the gene editing effects by activating the CRISPR system using engineered MPZ promoters. In this process, additional modification elements were considered to improve the expression efficiency of Cas9 protein or gRNA.

### Experimental Example 3-1. Specific example 1 of optimized additional expression system: Addition of poly A to protein of interest

The present inventors also sought to check the effects of the engineered MPZ promoters in other types of vectors with smaller sizes. Considering the packaging capacity of the AAV vector, vectors based on synthetic polyA (synpA, 49 bp) or simian virus 40 polyA (sv40pA, 104 bp, SEQ ID NO: 17), which were smaller in size compared to bovine growth hormone poly-A (BGHA, 225 bp) were used, and then the gene editing efficiency in the vectors was evaluated.

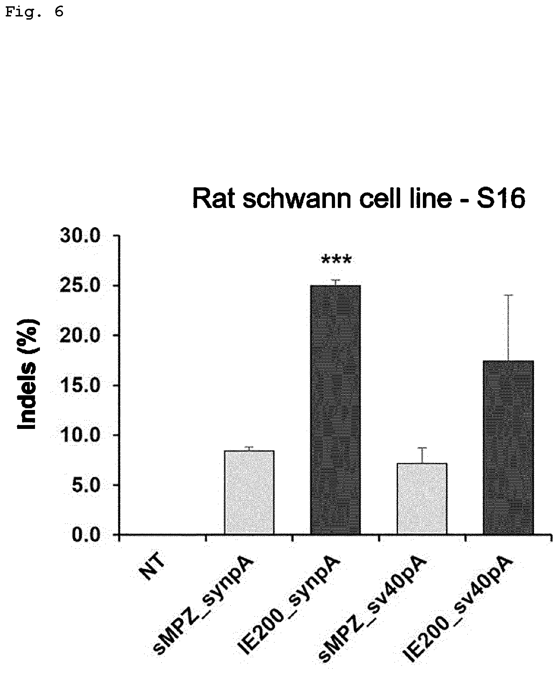

The results are shown in FIG. 6. FIG. 6 shows the results of verifying the gene editing efficiency for targeting the pmp22-TATA after co-transfection of the pAAV-SpCas9 expression vector and the pAAV-U6-sgRNA expression vector in S16 cells (N=3) . At this time, NT meant non-transfection; sMPZ mean a small MPZ core promoter (SEQ ID NO: 3); IE200 mean an engineered promoter (SEQ ID NO: 9); synpA mean synthetic poly-A (49 bp, SEQ ID NO: 18); and sv40pA mean simian virus 40 poly-A (104 bp, SEQ ID NO: 17). ***, p<0.001 vs sMPZ_synpA.

When using vectors based on synthetic polyA (synpA, 49 bp) or simian virus 40 polyA (sv40 pA, 104 bp, SEQ ID NO: 17), an increase in the efficiency of Cas9 expression by IE200 promoter was confirmed in S16 cells (FIG. 6). The experimental results show that the Schwann cell-specific promoters of the present disclosure may be suitable for use in small-sized vectors.

### Experimental Example 3-2. Specific example 2 of optimized additional expression system: Addition of WPRE (artificial intron) to protein of interest

Next, the effect of IE200 was additionally tested in RT4 cells using a SauriCas9-based vector, which was smaller than SpCas9 and might be produced as a single AAV.

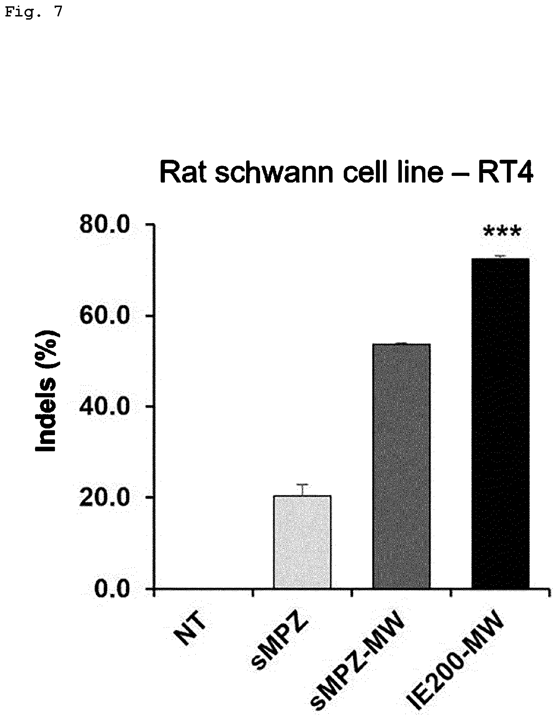

The results are shown in FIG. 7. FIG. 7 shows the results of verifying the gene editing efficiency for targeting pmp22-TATA after transfection of a single pAAV-Sauri-Cas9-U6-sgRNA expression vector in RT4 cells (N=3). At this time, NT meant non-transfection; sMPZ mean a small MPZ core promoter (SEQ ID NO: 3); IE200 mean an engineered promoter (SEQ ID NO: 9); and MW meant mvm intron + WPRE-short. ***, p<0.001 vs sMPZ-MW.

Compared to the sMPZ vector expressing SauriCas9 using sMPZ, when using the sMPZ-MW vector with added optimization elements such as mvm intron and WPRE, as shown in Table 1, the gene editing efficiency of pmp22-TATA targeted by SauriCas9 increased. The results show that the gene editing efficiency was significantly increased by the vector combining these optimization elements and the engineered MPZ promoters (FIG. 7). In particular, this shows that the IE200 promoter exhibited superior efficiency to other engineered MPZ promoters even when used in combination with various optimization elements. In addition, this demonstrates that all types of Cas9 that are applicable to Dual AAV or single AAV may be expressed by the engineered MPZ promoters in Schwann cells.

### Experimental Example 3-3. Specific example 3 of optimized additional expression system: gRNA expression vector with poly-T removed

Additional verification was conducted using IE200, which had the highest efficiency and the smallest size among engineered promoters, making it more compatible with AAV. In particular, research was conducted to confirm additional improvement effects when IE200 was used in combination with other optimization elements for gene editing. A vector such as the sf-modified vector was created by removing the poly-T from the sgRNA scaffold to enhance sgRNA expression driven by the U6 promoter, and then the vector was co-transfected into S16 cells for the experiments.

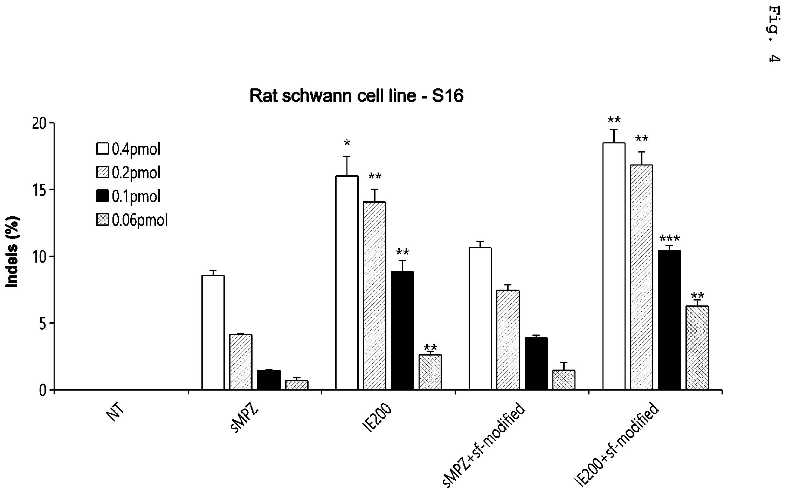

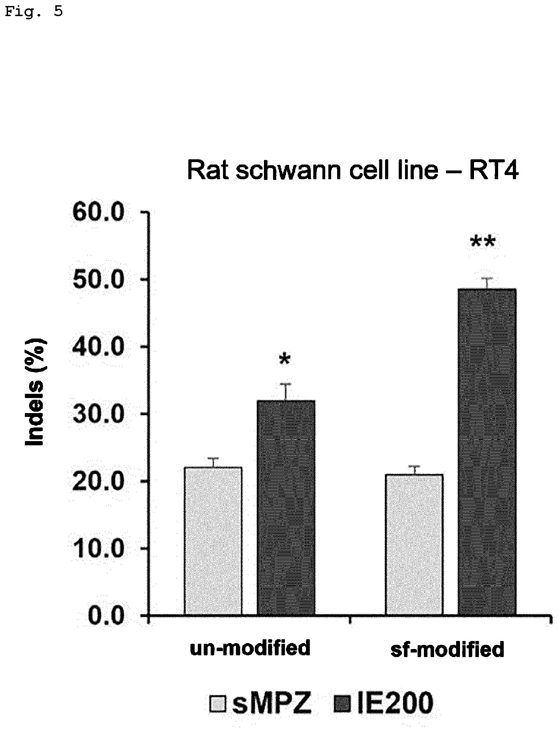

The results are shown in FIGs. 4 and 5. FIG. 4 shows the results of verifying the gene editing efficiency for targeting the pmp22-TATA after co-transfection of the pAAV-SpCas9-BHGA expression vector and the pAAV-U6-sf-modified-sgRNA expression vector in S16 cells (N=3-6) . FIG. 5 shows the results of verifying the gene editing efficiency for targeting the pmp22-TATA after co-transfection of the pAAV-SpCas9-BHGA expression vector and the pAAV-U6-sf-modified-sgRNA expression vector in RT4 cells (N=3-6). At this time, NT meant non-transfection; sMPZ mean a small MPZ core promoter (SEQ ID NO: 3); IE200 mean an engineered promoter (SEQ ID NO: 9); and Sf-modified meant the scaffold modification of sgRNA backbone. *, p<0.05 vs sMPZ (FIGs. 4 and 5) or sMPZ+sf-modified (FIG. 4); **, p<0.01 vs sMPZ (FIGs. 4 and 5) or sMPZ+sf-modified (FIG. 4); ***, p<0.001 vs sMPZ+sf-modified (FIG. 4).

As a result, it was confirmed that the gene editing efficiency was further increased under various dosage conditions when using the IE200 promoter together with the sf-modified vector (FIG. 4). This additional effect was similarly confirmed in RT4 cells, which was another type of rat Schwann cell (FIG. 5).

### Experimental Example 4. Effects of engineered MPZ promoters in experimental animals (rats)

Furthermore, the present inventors sought to confirm through animal experiments whether the promoters of the present disclosure had more specific effects on Schwann cells. In other words, it was confirmed whether the intravenously administered CRISPR composition might pass through tissues like the liver and exhibit more specific expression in nervous tissue.

After anesthetizing experimental animals (SD rats) with an anesthetic, reflex symptoms were checked to see whether the animals were anesthetized. Next, the anesthetized animals were placed in a calibration frame. The vector expressing sgRNA targeting the pmp22-TATA region (guide RNA1 in Table 1) were co-transfected into the experimental animals (rats) through the tail vein with a vector expressing SpCas9 under the control of the EFS promoter and a vector expressing SpCas9 under the control of IE200 promoter.

After 4 weeks, the animals were sacrificed, and the liver, sciatic nerve where Schwann cells were gathered, and lumbar nerve where Schwann cells were gathered were collected. Afterward, genomic DNA was prepared from the collected tissues or cells, and gene editing efficiency was tested.

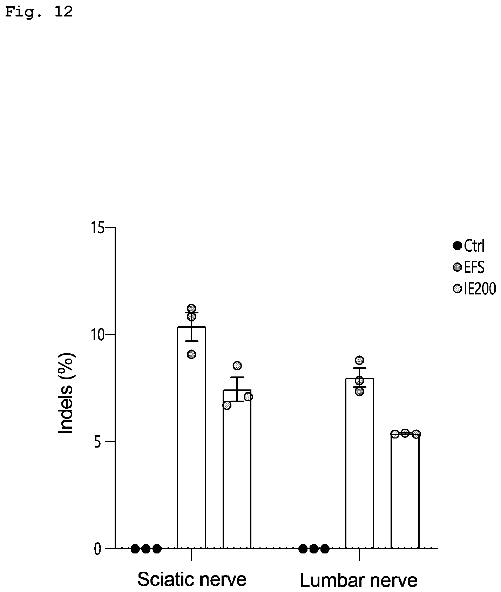

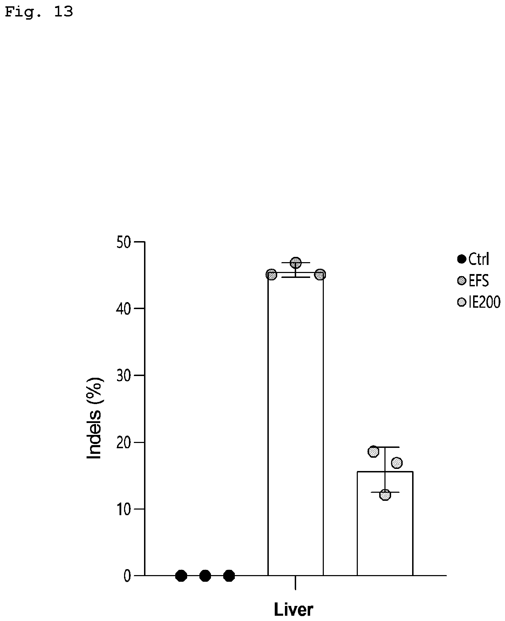

The results are shown in FIGs. 12 and 13. FIG. 12 shows the results of verifying the gene editing efficiency for targeting the pmp22-TATA in the sciatic nerve and lumbar nerve after injecting the pAAV-SpCas9-BHGA expression vector and the pAAV-U6-sgRNA expression vector into the tail vein of the rats. At this time, EFS was an EFS promoter (SEQ ID NO: 32); IE200 was an engineered promoter (SEQ ID NO: 9). FIG. 13 shows the results of verifying the gene editing efficiency for targeting the pmp22-TATA in the liver after injecting the pAAV-SpCas9-BHGA expression vector and the pAAV-U6-sgRNA expression vector into the tail vein of the rats. At this time, EFS was an EFS promoter (SEQ ID NO: 32); IE200 was an engineered promoter (SEQ ID NO: 9).

The present inventors sought to confirm whether the engineered MPZ promoter specifically expressed the protein of interest in nerve cells. In particular, hepatocytes were used as a control among cells other than nerve cells to confirm specific expression in nerve cells. This is because when with intravenous injection, the present inventors considered the fact that most of the administered substances have no choice but to pass through liver tissue in the systemic administration system.

Therefore, since the vectors were injected through the tail vein of rats in Experimental Example 6, it was expected that most of the injected vectors would move to the liver. Therefore, Cas9 expression and indel efficiency in the liver were expected to be sufficient even when promoters specific for nerve cells were used.

Therefore, the present inventors sought to determine whether the indel efficiency of the engineered MPZ promoter in nerve cells compared to liver cells was higher than that of other promoters, thereby confirming whether the promoter of the present disclosure was more specific to nerve cells. At this time, the EFS promoter, which was known to have a high expression rate in all tissues, was used as a promoter for comparison with the engineered MPZ promoter.

As a result, the EFS promoter showed a gene editing efficiency of close to 45% in liver tissue (FIG. 13) and a gene editing efficiency of approximately 10% in nerve cells (FIG. 12). In other words, nerve cells showed only about 1/5 the effect compared to liver cells.

In contrast, when IE200, which was the engineered MPZ promoter of the present disclosure, was used, the gene editing efficiency was about 15% in liver tissue (FIG. 13), and the efficiency was about 6% to 8% in nerve cells (FIG. 12). That is, nerve cells showed about half the effects compared to hepatocytes. In other words, this means that the engineered MPZ promoter of the present disclosure expressed the protein of interest more specifically in nerve cells.

These results show that when gene editing is necessary specifically for nerve cells, the engineered MPZ promoter is preferable to use than other promoters.

### Industrial Applicability

The present disclosure relates to an artificially manipulated minimal promoter that specifically operates in Schwann cells. The artificially manipulated minimal promoter is derived from the promoter of Myelin Protein Zero (MPZ or P0).

## Claims

1. A Schwann cell-specific promoter having a sequence selected from:
SEQ ID NO: 3; SEQ ID NO: 4; SEQ ID NO: 8; SEQ ID NO: 9; SEQ ID NO: 13; SEQ ID NO: 14; and a sequence having at least 90% or more homology with one of SEQ ID NOs: 3, 4, 8, 9, 13 and 14.

2. A Schwann cell-specific expression vector comprising:
a Schwann cell-specific promoter; and
a gene of interest,
wherein the Schwann cell-specific promoter has a sequence selected from a group consisting of SEQ ID NO: 3; SEQ ID NO: 4; SEQ ID NO: 8; SEQ ID NO: 9; SEQ ID NO: 13; SEQ ID NO: 14; and a sequence having at least 90% or more homology with one of SEQ ID NOs: 3, 4, 8, 9, 13 and 14,
wherein the gene of interest is a gene configured to be expressed in Schwann cells or a nucleic acid encoding a protein configured to be expressed in Schwann cells,
wherein the gene of interest is operably linked to the Schwann cell-specific promoter.

3. The Schwann cell-specific expression vector of claim 2,
wherein the Schwann cell-specific expression vector is a viral vector or non-viral vector.

4. The Schwann cell-specific expression vector of claim 3,
wherein the viral vector is one or more viral vector selected from a group consisting of retroviral (retrovirus) vector, lentiviral (lentivirus) vector, adenoviral (adenovirus) vector, adeno-associated viral (adeno-associated virus; AAV) vector, vaccinia viral (vaccinia virus) vector, poxviral (poxvirus) vector, and herpes simplex viral (herpes simplex virus) vector.

5. The Schwann cell-specific expression vector of claim 2,
wherein the Schwann cell-specific expression vector further comprises one or more regulatory/control element.

6. The Schwann cell-specific expression vector of claim 5,
wherein the regulatory/control element is an enhancer, artificial intron, polyadenylation signal and/or WPRE.

7. The Schwann cell-specific expression vector of claim 6,
wherein Schwann cell-specific expression vector comprises the polyadenylation signal,
wherein the polyadenylation signal is a synpA or SV40pA.

8. The Schwann cell-specific expression vector of claim 6,
wherein Schwann cell-specific expression vector comprises the artificial intron and the WPRE,
wherein the artificial intron is a MVM intron, and
wherein the WPRE is a WPRE3.

9. The Schwann cell-specific expression vector of claim 6, wherein Schwann cell-specific expression vector comprises the artificial intron, the WPRE, and the polyadenylation signal,
wherein the artificial intron is a MVM intron,
wherein the WPRE is a WPRE3, and
wherein the polyadenylation signal is synpA or SV40pA.

10. The Schwann cell-specific expression vector of claim 2,
wherein the gene of interest is a nucleic acid encoding a protein used for artificially manipulating genes in Schwann cell.

11. The Schwann cell-specific expression vector of claim 10,
wherein the protein for artificially manipulating genes is a Cas protein.

12. The Schwann cell-specific expression vector of claim 11,
wherein the Cas protein is Cas9 protein, Cas12a1 (Cpf1) protein, or Cas12f1 protein.

13. The Schwann cell-specific expression vector of claim 12,
wherein the Cas9 protein is a Streptococcus pyogenes Cas9 (SpCas9) protein, a Staphylococcus aureus Cas9 (SaCas9) protein, a Campylobacter jejuni Cas9 (CjCas9) protein, or a Staphylococcus auricularis Cas9 (SauriCas9) protein.

14. The Schwann cell-specific expression vector of claim 2,
wherein the gene of interest is a gene associated with treating (treatment of) a disease related to Schwann cell.

15. The Schwann cell-specific expression vector of claim 14,
wherein the gene associated with treating the disease related to Schwann cell is Cx32 (connexin 32) gene, SH3TC2 (SH3 domain and tetratricopeptide repeats 2) gene, MPZ (myelin protein zero) gene, EGR2 (early growth response 2) gene, GDAP1 (ganglioside induced differentiation associated protein 1) gene, NDRG1 (N-Myc downstream regulated 1) gene, or PMP22 (peripheral myelin protein 22) gene.

16. A composition comprising the expression vector of claim 10,
wherein the expression vector comprises a nucleic acid encoding Cas9 protein,
wherein the composition further comprises a guide RNA or a nucleic acid encoding the guide RNA.

17. The composition of claim 16,
wherein the guide RNA targets a gene that causes a disease related to Schwann cell,
wherein the gene that causes the disease related to Schwann cell is Cx32 (connexin 32) gene, SH3TC2 (SH3 domain and tetratricopeptide repeats 2) gene, MPZ (myelin protein zero) gene, EGR2 (early growth response 2) gene, GDAP1 (ganglioside induced differentiation associated protein 1) gene, NDRG1 (N-Myc downstream regulated 1) gene, or PMP22 (peripheral myelin protein 22) gene.
